# EUROPEAN PATENT APPLICATION

(11) **EP 4 644 550 A1**
(43) Date of publication of application: **05.11.2025**
(21) Application number: 24773947.7
(22) Date of filing: 11.03.2024
(51) Int. Cl.: C12N 15/45, A61K 39/155, A61K 39/12, C07K 14/005, C07K 14/135, A61K 48/00, A61P 31/14

(54) **VACCINE AGAINST RESPIRATORY SYNCYTIAL VIRUS INFECTION**

(30) Priority: 17.03.2023 CN 202310260673
(71) Applicant: WestVac Biopharma Co., Ltd., Chengdu, Sichuan 610000 (CN)
(72) Inventor: WEI, Xiawei, Chengdu, Sichuan 610065 (CN); LU, Guangwen, Chengdu, Sichuan 610065 (CN); CHENG, Ping, Chengdu, Sichuan 610065 (CN); YANG, Jingyun, Chengdu, Sichuan 610065 (CN); YANG, Li, Chengdu, Sichuan 610065 (CN); LI, Jiong, Chengdu, Sichuan 610065 (CN); WANG, Wei, Chengdu, Sichuan 610065 (CN); ZHAO, Zhiwei, Chengdu, Sichuan 610065 (CN); WEI, Yuquan, Chengdu, Sichuan 610065 (CN); YANG, Jinliang, Chengdu, Sichuan 610065 (CN); WANG, Zhenling, Chengdu, Sichuan 610065 (CN); SHEN, Guobo, Chengdu, Sichuan 610065 (CN)
(74) Representative: Gulde & Partner
(86) International application number: PCT/CN2024/081009
(87) International publication number: WO 2024/193380

(57) **Abstract**

Provided is a vaccine against respiration syncytial virus infection. In view of the lack of effective prevention and treatment drugs for respiratory syncytial virus infection for the elderly, infants, and people with low immunity, a vaccine against respiratory syncytial virus infection is provided. The vaccine is based on adenovirus as a vector, and the antigen gene expressed is optimized based on the sequence of respiratory syncytial virus F protein. The adenovirus vector vaccine can help a host resists respiratory syncytial virus infection and has a good preventive and therapeutic effect.

## Description

### TECHNICAL FIELD

The present invention relates to a vaccine against respiratory syncytial virus infection and pertains to the field of medicine.

### BACKGROUND

Respiratory syncytial virus (RSV) is the most important pathogen causing lower respiratory tract infection in children under 5 years old and the elderly worldwide. RSV belongs to the genus *pneumovirus* of paramyxoviridae, and is characterized as an antisense single-stranded RNA virus with only one serotype. It is mainly transmitted by contacting with virus-containing secretions or pollutants on nasopharyngeal mucosa or ocular mucosa. Scientists started the research of RSV vaccines in 1960s. However, inactivated RSV vaccines at that time not only failed to prevent RSV infection, but exacerbated illness severity in infants newly infected with RSV.

The virus is spherical in shape and is enveloped, with a diameter of 120 nm to 300 nm. A genome is characterized as a non-segmented single negative-stranded RNA and mainly encodes ten proteins, namely, three transmembrane proteins including fusion protein (F), small hydrophobic protein (SH) and adhesion protein (G), two matrix proteins M1 and M2, three proteins (L, N and P) that are bonded to viral RNA to form nucleocapsids, and two non-structural proteins (NS1 and NS2). A virus envelope has spikes composed of glycoprotein, without HA, NA and HL.

Fusion protein (F) is a surface-exposed viral envelope protein of RSV that mainly mediates viral entry into host cells, and has high sequence conservation, so it has become the main target protein for vaccine development. Protein F exists in two conformational states: pre-fusion (pre-F) and post-fusion (post-F). Infectious RSV displays both pre-fusion and post-fusion conformations of F protein on its surface, and the pre-F conformation is transformed into the post-F conformation after the cells are infected with virus. Since the virus was first discovered in 1956, its complex molecular structure and safety problems have hindered the development of vaccines.

### SUMMARY

To address the unmet need for effective prevention and treatment drugs against respiratory syncytial virus (RSV)-induced respiratory infection in the elderly, infants and immunocompromised persons, the present invention provides an adenovirus vector vaccine against respiratory syncytial virus infection; and the vaccine uses adenovirus as the vector, and F protein sequences of RSV are optimized and then designed as a series of pre-fusion (Pre-F) protein sequences, and the pre-fusion protein sequences are used as antigen genes and recombined into the adenovirus vector. Particularly, the antigen gene is designed based on the F protein of subtype A RSV, so that the adenovirus vector can express RSV antigen protein and help a host to resist respiratory syncytial virus infection, which has good prevention and treatment effect.

In a first aspect, the present invention provides an adenovirus vector vaccine for preventing and/or treating respiratory syncytial virus infection, where the adenovirus vector vaccine is obtained by constructing a recombinant adenovirus vector including a respiratory syncytial virus expression gene, and a polynucleotide sequence of the respiratory syncytial virus expression gene is selected from at least one of SEQ ID No.12, SEQ ID No.14, SEQ ID No.16, SEQ ID No.18, SEQ ID No.20, SEQ ID No.22, SEQ ID No.24, SEQ ID No.26, SEQ ID No.28, SEQ ID No.30, SEQ ID No.32 and SEQ ID No.34.

Further, an amino acid sequence of a protein encoded by the polynucleotide sequence is selected from at least one of SEQ ID No.11, SEQ ID No.13, SEQ ID No.15, SEQ ID No.17, SEQ ID No.19, SEQ ID No.21, SEQ ID No.23, SEQ ID No.25, SEQ ID No.27, SEQ ID No.29, SEQ ID NO.31 and SEQ ID No.33.

Further, the adenovirus vector vaccine further includes pharmaceutically acceptable adjuvants, vectors, diluents or excipients.

Further, the recombinant adenovirus vector is selected from at least one of the following: adenovirus, Ankara vaccinia virus and adeno-associated virus.

Preferably, the adenovirus vector is selected from replication-deficient adenovirus of human type 5, 35 or 26 or/and chimpanzee type AdC68 or AdC7.

More preferably, the adenovirus vector is selected from human type 5 replication-deficient adenovirus with combined deletion of E1 and E3.

Further, the adenovirus vector vaccine is formulated as an intradermal injection preparation or a subcutaneous injection preparation, an intramuscular injection preparation, an intravenous injection preparation, an oral preparation or a nasal spray preparation.

Preferably, the vaccine is formulated as the nasal spray preparation or the intramuscular injection preparation.

The present invention provides a method for preparing an adenovirus in the adenovirus vector vaccine, and the method includes the following steps: constructing a shuttle plasmid vector comprising polynucleotide; transfecting the constructed shuttle plasmid vector and backbone plasmids into a host cell to culture the host cell; obtaining a replication-deficient recombinant adenovirus; and performing large-scale cultivation and purification.
SEQ ID No.11
   RSV-A-PreF-1 (R1) (Del104-145aa) 
MELLILKANAITTILTAVTFCFASGQNITEEFYQSTCSAVSKGYLSALRTGWYTSVITI
ELSNIKENKCNGTDAKVKLIKQELDKYKNAVTELQLLMQSTPPTSAIASGVAVSKVLHLE
GEVNKIKSALLSTNKAVVSLSNGVSVLTSKVLDLKNYIDKQLLPIVNKQSCSISNIETVIEF
QQKNNRLLEITREFSVNAGVTTPVSTYMLTNSELLSLINDMPITNDQKKLMSNNVQIVRQ
QSYSIMSIIKEEVLAYVVQLPLYGVIDTPCWKLHTSPLCTTNTKEGSNICLTRTDRGWYCD
NAGSVSFFPQAETCKVQSNRVFCDTMNSLTLPSEINLCNVDIFNPKYDCKIMTSKTDVSS
SVITSLGAIVSCYGKTKCTASNKNRGIIKTFSNGCDYVSNKGMDTVSVGNTLYYVNKQE
GKSLYVKGEPIINFYDPLVFPSDEFDASISQVNEKINQSLAFIRKSDELLSAIGGYIPEAPRD
GQAYVRKDGEWVLLSTFL
SEQ ID No. 12 encoding polypeptide of SEQ ID No. 11 
ATGGAGCTGCTGATCCTGAAGGCCAACGCCATCACCACCATCCTGACCGCCGTG
ACCTTCTGCTTCGCTAGCGGGCAGAACATCACCGAGGAGTTCTATCAGAGCACCTGC
AGCGCTGTGAGCAAAGGCTATCTGAGCGCCCTGAGAACCGGCTGGTACACAAGCGT
CATCACCATCGAGCTGAGCAACATCAAGGAGAACAAGTGCAACGGCACCGACGCCA
AGGTGAAGCTGATCAAGCAAGAGCTGGACAAGTACAAGAACGCCGTGACCGAGCTG
CAGCTCCTGATGCAGAGCACCCCCCCTACCTCCGCTATTGCTAGCGGCGTGGCCGTGA
GCAAGGTCCTGCACCTGGAGGGCGAGGTGAACAAGATCAAGAGCGCCCTGCTCAGC
ACCAACAAGGCCGTGGTGAGCCTGAGCAACGGCGTGAGCGTGCTGACAAGCAAGGT
GCTGGACCTGAAGAACTACATCGACAAGCAGCTGCTGCCCATCGTGAACAAGCAGA
GCTGCAGCATCAGCAACATCGAGACCGTGATCGAGTTTCAGCAGAAGAACAACAGA
CTGCTGGAGATCACAAGAGAGTTCAGCGTGAACGCCGGCGTGACCACCCCCGTGAG
 
CACCTACATGCTGACCAACAGCGAACTGCTGTCCCTGATTAACGACATGCCCATCACC
AACGATCAGAAGAAGCTGATGAGCAACAACGTGCAGATCGTGAGACAGCAGAGCTA
CAGCATCATGAGCATCATCAAGGAAGAGGTGCTGGCCTACGTGGTGCAGCTGCCCCT
GTACGGCGTGATCGACACCCCCTGCTGGAAGCTGCACACAAGCCCCCTGTGCACCAC
CAACACCAAGGAGGGCAGCAACATCTGCCTGACAAGAACCGACAGAGGCTGGTACT
GCGACAACGCCGGCAGCGTGAGCTTCTTCCCCCAAGCCGAGACCTGCAAGGTGCAG
AGCAACAGAGTGTTCTGCGACACCATGAACAGCCTGACCCTGCCTAGCGAGATCAAC
CTGTGCAACGTGGACATCTTCAACCCCAAGTACGACTGCAAGATCATGACAAGCAAG
ACCGACGTGAGCAGCAGCGTGATCACAAGCCTGGGCGCCATCGTGAGCTGCTACGGC
AAGACCAAGTGCACCGCTAGCAACAAGAACAGAGGCATCATCAAGACCTTCAGCAA
CGGCTGCGACTACGTGAGCAACAAGGGCATGGACACCGTGAGCGTGGGCAACACCC
TGTACTACGTCAACAAGCAAGAGGGGAAGAGCCTGTACGTGAAGGGCGAGCCCATC
ATCAACTTCTACGACCCCCTGGTGTTCCCTAGCGACGAGTTCGACGCTAGCATCAGCC
AAGTGAACGAGAAGATCAATCAGAGCCTGGCCTTCATCAGAAAGAGCGACGAGCTG
CTGAGCGCTATCGGCGGCTACATCCCCGAGGCCCCTAGAGACGGCCAAGCCTACGTG
AGAAAGGACGGCGAGTGGGTGCTGCTGTCCACCTTCCTCTGA
SEQ ID No.13
   RSV-A-PreF-1 (R2) (Del104-145aa) 
MELLILKANAITTILTAVTFCFASGQNITEEFYQSTCSAVSKGYLSALRTGWYTSVITI
ELSNIKENKCNGTDAKVKLIKQELDKYKNAVTELQLLMQSTPPTSAIASGVAVSKVLHLE
GEVNKIKSALLSTNKAVVSLSNGVSVLTSKVLDLKNYIDKQLLPIVNKQSCSISNIETVIEF
QQKNNRLLEITREFSVNAGVTTPVSTYMLTNSELLSLINDMPITNDQKKLMSNNVQIVRQ
QSYSIMSIIKEEVLAYVVQLPLYGVIDTPCWKLHTSPLCTTNTKEGSNICLTRTDRGWYCD
NAGSVSFFPQAETCKVQSNRVFCDTMNSLTLPSEINLCNVDIFNPKYDCKIMTSKTDVSS
SVITSLGAIVSCYGKTKCTASNKNRGIIKTFSNGCDYVSNKGMDTVSVGNTLYYVNKQE
GKSLYVKGEPIINFYDPLVFPSDEFDASISQVNEKINQSLAFIRKSDELLHNVNAGKSTTNI
MITTIIIVIIVILLSLIAVGLLLYCKARSTPVTLSKDQLSGINNIAFSN
SEQ ID No. 14 encoding polypeptide of SEQ ID No. 13 
ATGGAGCTGCTGATCCTGAAGGCCAACGCCATCACAACCATCCTGACCGCCGTG
ACCTTCTGCTTCGCTAGCGGGCAGAACATCACCGAGGAGTTCTATCAGAGCACCTGC
AGCGCCGTGAGCAAGGGGTATCTGAGCGCCCTGAGAACCGGCTGGTACACCTCCGTC
 
ATCACCATCGAGCTGAGCAACATCAAGGAGAACAAGTGCAACGGCACCGACGCCAA
GGTGAAGCTGATCAAGCAAGAGCTGGACAAGTACAAGAACGCCGTGACCGAGCTGC
AACTGCTGATGCAAAGCACCCCTCCCACAAGCGCCATCGCCTCCGGCGTGGCTGTCA
GCAAGGTCCTCCATCTGGAGGGCGAGGTGAACAAGATCAAGAGCGCCCTGCTGAGC
ACCAACAAGGCCGTGGTGAGCCTGAGCAACGGCGTGAGCGTGCTGACCTCCAAGGT
GCTGGATCTGAAGAACTACATCGACAAGCAGCTGCTGCCCATCGTGAACAAGCAGAG
CTGCAGCATCAGCAACATCGAGACCGTGATCGAGTTTCAGCAGAAGAACAACAGAC
TGCTGGAGATCACAAGAGAGTTCAGCGTGAATGCTGGCGTCACCACCCCCGTGAGCA
CCTACATGCTGACCAACAGCGAGCTGCTGAGCCTGATCAACGACATGCCCATCACCA
ACGATCAGAAGAAGCTGATGAGCAACAACGTGCAGATCGTGAGACAGCAGAGCTAC
AGCATCATGAGCATCATCAAGGAAGAGGTGCTGGCCTACGTGGTGCAGCTGCCCCTG
TACGGCGTGATCGACACCCCCTGCTGGAAGCTGCACACAAGCCCCCTGTGCACAACC
AACACCAAGGAGGGCAGCAACATCTGCCTGACAAGAACCGACAGAGGCTGGTACTG
CGACAACGCCGGCAGCGTGAGCTTCTTCCCCCAAGCCGAGACCTGCAAGGTGCAGA
GCAACAGAGTGTTCTGCGACACCATGAACAGCCTGACCCTGCCTAGCGAGATCAACC
TGTGCAACGTGGACATCTTCAACCCCAAGTACGACTGCAAGATCATGACAAGCAAGA
CCGACGTGAGCAGCAGCGTGATTACAAGCCTGGGCGCCATCGTGAGCTGCTACGGCA
AGACCAAGTGCACCGCTAGCAACAAGAACAGAGGCATCATCAAGACCTTCAGCAAC
GGCTGCGACTACGTGAGCAACAAGGGCATGGACACCGTGAGCGTGGGCAACACCCT
GTACTACGTGAATAAGCAAGAGGGCAAGAGCCTGTACGTGAAGGGCGAGCCCATCAT
CAACTTCTACGACCCCCTGGTGTTCCCTAGCGACGAGTTCGACGCTAGCATCAGCCA
AGTGAACGAGAAGATCAATCAGAGCCTGGCCTTCATCAGAAAGAGCGACGAGCTGC
TGCACAACGTGAACGCCGGCAAGAGCACCACCAACATCATGATCACCACCATCATTA
TCGTGATCATCGTGATTCTCCTGTCCCTGATCGCCGTCGGCCTGCTCCTGTACTGCAAG
GCTAGAAGCACCCCCGTGACCCTGAGCAAGGATCAGCTGAGCGGCATCAACAACATC
GCCTTCAGCAACTGA
SEQ ID No.15
   RSV-A-PreF-1 (R3) (2 disulfide bond mutations) 
MELLILKANAITTILTAVTFCFASGQNCTEEFYQSTCSAVSKGYLSALRTGWYTCVITI
ELSNIKENKCNGTDAKVKLIKQELDKYKNAVTELQLLMQSTPPTNNRARRELPRFMNYT
LNNAKKTNVTLSKKRKRRFLGFLLGVGSAIASGVAVSKVLHLEGEVNKIKSALLSTNKA
 
VVSLSNGVSVCTSKVLDLKNYIDKQLLPIVNKQSCSISNIETVIEFQQKNNRLLEITREFSV
NAGVTTPVSTYMLTNSELLSLINDMPITNDQKKLMSNNVQIVRQQSYSIMSIIKEEVLAY
VVQLPLYGVIDTPCWKLHTSPLCTTNTKEGSNICLTRTDRGWYCDNAGSVSFFPQAETC
KVQSNRVFCDTMNSLTLPSEINLCNVDIFNPKYDCKIMTSKTDVSSSVITSLGAIVSCYGK
TKCTASNKNRGIIKTFSNGCDYVSNKGMDTVSVGNTLYYVNKQECKSLYVKGEPIINFY
DPLVFPSDEFDASISQVNEKINQSLAFIRKSDELLSAIGGYIPEAPRDGQAYVRKDGEWVL
LSTFL
SEQ ID No. 16 encoding polypeptide of SEQ ID No. 15 
ATGGAGCTGCTGATCCTGAAGGCCAACGCCATCACCACCATCCTGACCGCCGTG
ACCTTCTGCTTCGCTAGCGGGCAGAACTGCACCGAGGAGTTCTATCAGAGCACCTGC
AGCGCCGTCAGCAAAGGCTACCTGAGCGCCCTGAGAACCGGCTGGTACACCTGCGT
GATTACCATCGAGCTGAGCAACATCAAGGAGAACAAGTGCAACGGCACCGACGCCA
AGGTGAAGCTGATCAAGCAAGAGCTGGACAAGTACAAGAACGCCGTGACCGAGCTG
CAGCTGCTGATGCAGAGCACCCCCCCCACCAACAACAGAGCTAGAAGAGAGCTGCC
TAGATTCATGAACTACACCCTGAACAACGCCAAGAAGACCAACGTGACCCTGAGCAA
GAAGAGAAAGAGAAGATTCCTGGGCTTCCTGCTGGGCGTGGGCAGCGCCATTGCTAG
CGGCGTGGCCGTGAGCAAGGTGCTGCACCTCGAGGGCGAGGTGAACAAGATCAAGA
GCGCCCTGCTGTCCACCAACAAGGCCGTGGTGAGCCTGAGCAACGGCGTGAGCGTG
TGCACAAGCAAGGTCCTGGACCTGAAGAACTACATCGACAAGCAGCTGCTGCCCATC
GTCAACAAGCAATCCTGCAGCATCAGCAACATCGAGACCGTGATCGAGTTTCAGCAG
AAGAACAACAGACTGCTGGAGATCACAAGAGAGTTCAGCGTGAACGCCGGCGTGAC
CACCCCCGTGAGCACCTACATGCTGACCAACAGCGAACTCCTGAGCCTGATCAACGA
CATGCCCATCACCAACGATCAGAAGAAGCTGATGAGCAACAACGTGCAGATCGTGAG
ACAGCAGAGCTACAGCATCATGAGCATCATCAAGGAAGAGGTGCTGGCCTACGTGGT
GCAGCTGCCCCTGTACGGCGTGATCGACACCCCCTGCTGGAAGCTGCACACAAGCCC
CCTGTGCACCACCAACACCAAGGAGGGCAGCAACATCTGCCTGACAAGAACCGACA
GAGGCTGGTACTGCGACAACGCCGGCAGCGTGAGCTTCTTCCCCCAAGCCGAGACCT
GCAAGGTGCAGAGCAACAGAGTGTTCTGCGACACCATGAACAGCCTGACCCTGCCT
AGCGAGATCAACCTGTGCAACGTGGACATCTTCAACCCCAAGTACGACTGCAAGATC
ATGACAAGCAAGACCGACGTGAGCAGCAGCGTGATCACAAGCCTGGGCGCCATCGT
GAGCTGCTACGGCAAGACCAAGTGCACCGCTAGCAACAAGAACAGAGGCATCATCA
 
AGACCTTCAGCAACGGCTGCGACTACGTGAGCAACAAGGGCATGGACACCGTGAGC
GTGGGCAACACCCTGTACTACGTGAATAAACAAGAGTGCAAGAGCCTGTACGTGAAG
GGCGAGCCCATCATCAACTTCTACGACCCCCTGGTGTTCCCTAGCGACGAGTTCGAC
GCTAGCATCAGCCAAGTGAACGAGAAGATCAATCAGAGCCTGGCCTTCATCAGAAAG
AGCGACGAGCTGCTGAGCGCCATCGGCGGCTACATCCCCGAGGCCCCTAGAGACGGC
CAAGCCTACGTGAGAAAGGACGGCGAGTGGGTGCTGCTGAGCACCTTCCTGTGA
SEQ ID No.17
   RSV-A-PreF-1 (R4) (2 disulfide bond mutations) 
MELLILKANAITTILTAVTFCFASGQNCTEEFYQSTCSAVSKGYLSALRTGWYTCVITI
ELSNIKENKCNGTDAKVKLIKQELDKYKNAVTELQLLMQSTPPTNNRARRELPRFMNYT
LNNAKKTNVTLSKKRKRRFLGFLLGVGSAIASGVAVSKVLHLEGEVNKIKSALLSTNKA
VVSLSNGVSVCTSKVLDLKNYIDKQLLPIVNKQSCSISNIETVIEFQQKNNRLLEITREFSV
NAGVTTPVSTYMLTNSELLSLINDMPITNDQKKLMSNNVQIVRQQSYSIMSIIKEEVLAY
VVQLPLYGVIDTPCWKLHTSPLCTTNTKEGSNICLTRTDRGWYCDNAGSVSFFPQAETC
KVQSNRVFCDTMNSLTLPSEINLCNVDIFNPKYDCKIMTSKTDVSSSVITSLGAIVSCYGK
TKCTASNKNRGIIKTFSNGCDYVSNKGMDTVSVGNTLYYVNKQECKSLYVKGEPIINFY
DPLVFPSDEFDASISQVNEKINQSLAFIRKSDELLHNVNAGKSTTNIMITTIIIVIIVILLSLIA
VGLLLYCKARSTPVTLSKDQLSGINNIAFSN
SEQ ID No.18 encoding polynucleotide of SEQ ID No.17 
ATGGAGCTGCTGATCCTGAAGGCCAACGCCATCACCACCATCCTGACCGCCGTG
ACCTTCTGCTTCGCTAGCGGGCAGAACTGCACCGAGGAGTTCTATCAGAGCACCTGC
AGCGCCGTGAGCAAGGGCTACCTGAGCGCCCTGAGAACCGGCTGGTACACCTGTGT
CATCACCATCGAGCTGAGCAACATCAAGGAGAACAAGTGCAACGGCACCGACGCCA
AGGTGAAGCTGATCAAGCAAGAGCTGGACAAGTACAAGAACGCCGTGACCGAGCTG
CAGCTGCTGATGCAGAGCACCCCCCCCACCAACAACAGAGCTAGAAGAGAGCTGCC
TAGATTCATGAACTACACCCTGAACAACGCCAAGAAGACCAACGTGACCCTGAGCAA
GAAAAGAAAGAGAAGATTCCTGGGGTTCCTGCTGGGGGTGGGCAGCGCCATTGCTA
GCGGCGTGGCCGTCTCCAAGGTCCTGCACCTGGAGGGCGAGGTGAACAAGATCAAG
AGCGCCCTGCTGAGCACCAACAAGGCCGTGGTGAGCCTGAGCAACGGCGTGAGCGT
GTGCACAAGCAAGGTGCTGGACCTGAAGAACTACATCGACAAGCAGCTGCTGCCCAT
CGTCAACAAACAGTCCTGCAGCATCAGCAACATCGAGACCGTGATCGAGTTTCAGCA
 
GAAGAACAACAGACTGCTGGAGATCACAAGAGAGTTCAGCGTCAATGCCGGGGTGA
CCACCCCCGTGAGCACCTACATGCTGACCAACAGCGAGCTGCTGAGCCTCATCAACG
ACATGCCCATCACCAACGATCAGAAGAAGCTGATGAGCAACAACGTGCAGATCGTGA
GACAGCAGAGCTACAGCATCATGAGCATCATCAAGGAAGAGGTGCTGGCCTACGTGG
TGCAGCTGCCCCTGTACGGCGTGATCGACACCCCCTGCTGGAAGCTGCACACAAGCC
CCCTGTGTACCACCAACACCAAAGAGGGCAGCAACATCTGCCTGACAAGAACCGAC
AGAGGCTGGTACTGCGACAACGCCGGCAGCGTGAGCTTCTTCCCCCAAGCCGAGAC
CTGCAAGGTGCAGAGCAACAGAGTGTTCTGCGACACCATGAACAGCCTGACCCTGC
CTAGCGAGATCAACCTGTGCAACGTGGACATCTTCAACCCCAAGTACGACTGCAAGA
TCATGACAAGCAAGACCGACGTGAGCAGCTCCGTGATCACAAGCCTGGGCGCCATCG
TGAGCTGCTACGGCAAGACCAAGTGCACCGCTAGCAACAAGAACAGAGGCATCATC
AAGACCTTCAGCAACGGCTGCGACTACGTGAGCAACAAGGGCATGGACACCGTGAG
CGTGGGCAACACCCTGTACTACGTGAACAAGCAAGAGTGCAAGAGCCTGTACGTGA
AGGGCGAGCCCATCATCAACTTCTACGACCCCCTGGTGTTCCCTAGCGACGAGTTCG
ACGCTAGCATCAGCCAAGTGAACGAGAAGATCAATCAGAGCCTGGCCTTCATCAGAA
AGAGCGACGAGCTGCTGCACAACGTGAACGCCGGCAAGAGCACCACCAACATCATG
ATCACAACCATCATTATCGTGATCATCGTGATTCTCCTGAGCCTGATCGCCGTGGGCCT
GCTCCTGTACTGCAAGGCTAGAAGCACCCCCGTGACCCTGAGCAAGGATCAGCTGAG
CGGCATCAACAACATCGCCTTCAGCAACTGA
SEQ ID No.19 
MELLILKANAITTILTAVTFCFASGQNCTEEFYQSTCSAVSKGYLSALRTGWYTSVITI
ELSNIKENKCNGTDAKVKLIKQELDKYKNAVTELQLLMQSTPPTSAIASGVAVSKVLHLE
GEVNKIKSALLSTNKAVVSLSNGVSVLTSKVLDLKNYIDKQLLPIVNKQSCSISNIETVIEF
QQKNNRLLEITREFSVNAGVTTPVSTYMLTNSELLSLINDMPITNDQKKLMSNNVQIVRQ
QSYSIMSIIKEEVLAYVVQLPLYGVIDTPCWKLHTSPLCTTNTKEGSNICLTRTDRGWYCD
NAGSVSFFPQAETCKVQSNRVFCDTMNSLTLPSEINLCNVDIFNPKYDCKIMTSKTDVSS
SVITSLGAIVSCYGKTKCTASNKNRGIIKTFSNGCDYVSNKGMDTVSVGNTLYYVNKQE
CKSLYVKGEPIINFYDPLVFPSDEFDASISQVNEKINQSLAFIRKSDELLSAIGGYIPEAPRD
GQAYVRKDGEWVLLSTFL
SEQ ID No.20 encoding polynucleotide of SEQ ID No.19 
ATGGAGCTGCTGATCCTGAAGGCCAACGCCATCACCACCATCCTGACCGCCGTG
ACCTTCTGCTTCGCTAGCGGGCAGAACTGCACCGAGGAGTTCTATCAGAGCACCTGC
AGCGCTGTGAGCAAAGGCTATCTGAGCGCCCTGAGAACCGGCTGGTACACAAGCGT
CATCACCATCGAGCTGAGCAACATCAAGGAGAACAAGTGCAACGGCACCGACGCCA
AGGTGAAGCTGATCAAGCAAGAGCTGGACAAGTACAAGAACGCCGTGACCGAGCTG
CAGCTCCTGATGCAGAGCACCCCCCCTACCTCCGCTATTGCTAGCGGCGTGGCCGTGA
GCAAGGTCCTGCACCTGGAGGGCGAGGTGAACAAGATCAAGAGCGCCCTGCTCAGC
ACCAACAAGGCCGTGGTGAGCCTGAGCAACGGCGTGAGCGTGCTGACAAGCAAGGT
GCTGGACCTGAAGAACTACATCGACAAGCAGCTGCTGCCCATCGTGAACAAGCAGA
GCTGCAGCATCAGCAACATCGAGACCGTGATCGAGTTTCAGCAGAAGAACAACAGA
CTGCTGGAGATCACAAGAGAGTTCAGCGTGAACGCCGGCGTGACCACCCCCGTGAG
CACCTACATGCTGACCAACAGCGAACTGCTGTCCCTGATTAACGACATGCCCATCACC
AACGATCAGAAGAAGCTGATGAGCAACAACGTGCAGATCGTGAGACAGCAGAGCTA
CAGCATCATGAGCATCATCAAGGAAGAGGTGCTGGCCTACGTGGTGCAGCTGCCCCT
GTACGGCGTGATCGACACCCCCTGCTGGAAGCTGCACACAAGCCCCCTGTGCACCAC
CAACACCAAGGAGGGCAGCAACATCTGCCTGACAAGAACCGACAGAGGCTGGTACT
GCGACAACGCCGGCAGCGTGAGCTTCTTCCCCCAAGCCGAGACCTGCAAGGTGCAG
AGCAACAGAGTGTTCTGCGACACCATGAACAGCCTGACCCTGCCTAGCGAGATCAAC
CTGTGCAACGTGGACATCTTCAACCCCAAGTACGACTGCAAGATCATGACAAGCAAG
ACCGACGTGAGCAGCAGCGTGATCACAAGCCTGGGCGCCATCGTGAGCTGCTACGGC
AAGACCAAGTGCACCGCTAGCAACAAGAACAGAGGCATCATCAAGACCTTCAGCAA
CGGCTGCGACTACGTGAGCAACAAGGGCATGGACACCGTGAGCGTGGGCAACACCC
TGTACTACGTCAACAAGCAAGAGTGTAAGAGCCTGTACGTGAAGGGCGAGCCCATCA
TCAACTTCTACGACCCCCTGGTGTTCCCTAGCGACGAGTTCGACGCTAGCATCAGCCA
AGTGAACGAGAAGATCAATCAGAGCCTGGCCTTCATCAGAAAGAGCGACGAGCTGC
TGAGCGCTATCGGCGGCTACATCCCCGAGGCCCCTAGAGACGGCCAAGCCTACGTGA
GAAAGGACGGCGAGTGGGTGCTGCTGTCCACCTTCCTCTGA
SEQ ID No.21
   RSV-A-PreF-3F (R6) (Del104-145aa+1 disulfide bond mutation) 
MELLILKANAITTILTAVTFCFASGQNCTEEFYQSTCSAVSKGYLSALRTGWYTSVITI
ELSNIKENKCNGTDAKVKLIKQELDKYKNAVTELQLLMQSTPPTSAIASGVAVSKVLHLE
 
GEVNKIKSALLSTNKAVVSLSNGVSVLTSKVLDLKNYIDKQLLPIVNKQSCSISNIETVIEF
QQKNNRLLEITREFSVNAGVTTPVSTYMLTNSELLSLINDMPITNDQKKLMSNNVQIVRQ
QSYSIMSIIKEEVLAYVVQLPLYGVIDTPCWKLHTSPLCTTNTKEGSNICLTRTDRGWYCD
NAGSVSFFPQAETCKVQSNRVFCDTMNSLTLPSEINLCNVDIFNPKYDCKIMTSKTDVSS
SVITSLGAIVSCYGKTKCTASNKNRGIIKTFSNGCDYVSNKGMDTVSVGNTLYYVNKQE
CKSLYVKGEPIINFYDPLVFPSDEFDASISQVNEKINQSLAFIRKSDELLHNVNAGKSTTNI
MITTIIIVIIVILLSLIAVGLLLYCKARSTPVTLSKDQLSGINNIAFSN
SEQ ID No.22 encoding polynucleotide of SEQ ID No.21 
ATGGAGCTGCTGATCCTGAAGGCCAACGCCATCACAACCATCCTGACCGCCGTG
ACCTTCTGCTTCGCTAGCGGGCAGAACTGCACCGAGGAGTTCTATCAGAGCACCTGC
AGCGCCGTGAGCAAGGGGTATCTGAGCGCCCTGAGAACCGGCTGGTACACCTCCGTC
ATCACCATCGAGCTGAGCAACATCAAGGAGAACAAGTGCAACGGCACCGACGCCAA
GGTGAAGCTGATCAAGCAAGAGCTGGACAAGTACAAGAACGCCGTGACCGAGCTGC
AACTGCTGATGCAAAGCACCCCTCCCACAAGCGCCATCGCCTCCGGCGTGGCTGTCA
GCAAGGTCCTCCATCTGGAGGGCGAGGTGAACAAGATCAAGAGCGCCCTGCTGAGC
ACCAACAAGGCCGTGGTGAGCCTGAGCAACGGCGTGAGCGTGCTGACCTCCAAGGT
GCTGGATCTGAAGAACTACATCGACAAGCAGCTGCTGCCCATCGTGAACAAGCAGAG
CTGCAGCATCAGCAACATCGAGACCGTGATCGAGTTTCAGCAGAAGAACAACAGAC
TGCTGGAGATCACAAGAGAGTTCAGCGTGAATGCTGGCGTCACCACCCCCGTGAGCA
CCTACATGCTGACCAACAGCGAGCTGCTGAGCCTGATCAACGACATGCCCATCACCA
ACGATCAGAAGAAGCTGATGAGCAACAACGTGCAGATCGTGAGACAGCAGAGCTAC
AGCATCATGAGCATCATCAAGGAAGAGGTGCTGGCCTACGTGGTGCAGCTGCCCCTG
TACGGCGTGATCGACACCCCCTGCTGGAAGCTGCACACAAGCCCCCTGTGCACAACC
AACACCAAGGAGGGCAGCAACATCTGCCTGACAAGAACCGACAGAGGCTGGTACTG
CGACAACGCCGGCAGCGTGAGCTTCTTCCCCCAAGCCGAGACCTGCAAGGTGCAGA
GCAACAGAGTGTTCTGCGACACCATGAACAGCCTGACCCTGCCTAGCGAGATCAACC
TGTGCAACGTGGACATCTTCAACCCCAAGTACGACTGCAAGATCATGACAAGCAAGA
CCGACGTGAGCAGCAGCGTGATTACAAGCCTGGGCGCCATCGTGAGCTGCTACGGCA
AGACCAAGTGCACCGCTAGCAACAAGAACAGAGGCATCATCAAGACCTTCAGCAAC
GGCTGCGACTACGTGAGCAACAAGGGCATGGACACCGTGAGCGTGGGCAACACCCT
GTACTACGTGAATAAGCAAGAGTGCAAGAGCCTGTACGTGAAGGGCGAGCCCATCAT
 
CAACTTCTACGACCCCCTGGTGTTCCCTAGCGACGAGTTCGACGCTAGCATCAGCCA
AGTGAACGAGAAGATCAATCAGAGCCTGGCCTTCATCAGAAAGAGCGACGAGCTGC
TGCACAACGTGAACGCCGGCAAGAGCACCACCAACATCATGATCACCACCATCATTA
TCGTGATCATCGTGATTCTCCTGTCCCTGATCGCCGTCGGCCTGCTCCTGTACTGCAAG
GCTAGAAGCACCCCCGTGACCCTGAGCAAGGATCAGCTGAGCGGCATCAACAACATC
GCCTTCAGCAACTGA
SEQ ID No.23
   RSV-B-PreF-1 (R7) (Del104-145aa) 
MELLIHRSSAIFLTLAVNALYLTSSQNITEEFYQSTCSAVSRGYFSALRTGWYTSVITIE
LSNIKETKCNGTDTKVKLIKQELDKYKNAVTELQLLMQNTPAASAIASGIAVSKVLHLEG
EVNKIKNALLSTNKAVVSLSNGVSVLTSKVLDLKNYINNRLLPIVNQQSCRISNIETVIEF
QQMNSRLLEITREFSVNAGVTTPLSTYMLTNSELLSLINDMPITNDQKKLMSSNVQIVRQ
QSYSIMSIIKEEVLAYVVQLPIYGVIDTPCWKLHTSPLCTTNIKEGSNICLTRTDRGWYCD
NAGSVSFFPQADTCKVQSNRVFCDTMNSLTLPSEVSLCNTDIFNSKYDCKIMTSKTDISSS
VITSLGAIVSCYGKTKCTASNKNRGIIKTFSNGCDYVSNKGVDTVSVGNTLYYVNKLEG
KNLYVKGEPIINYYDPLVFPSDEFDASISQVNEKINQSLAFIRRSDELLSAIGGYIPEAPRDG
QAYVRKDGEWVLLSTFL
SEQ ID No.24 encoding polynucleotide of SEQ ID No.23 
ATGGAGCTGCTGATCCACAGAAGCAGCGCCATCTTCCTGACCCTGGCCGTGAAC
GCCCTGTACCTGACAAGCAGCCAAAACATCACCGAGGAGTTCTATCAGAGCACCTGC
AGCGCCGTGAGCAGAGGCTACTTCAGCGCCCTGAGAACCGGCTGGTACACCTCCGTG
ATCACAATCGAACTGAGCAACATTAAGGAGACCAAGTGTAACGGCACCGACACCAA
GGTGAAGCTGATCAAGCAAGAGCTGGACAAGTACAAGAACGCCGTGACCGAGCTGC
AGCTGCTGATGCAGAACACCCCCGCCGCTAGCGCCATCGCTAGCGGCATCGCCGTGT
CCAAAGTGCTGCACCTGGAGGGCGAGGTGAACAAGATCAAGAACGCCCTGCTGAGC
ACAAACAAGGCCGTGGTGAGCCTGAGCAACGGCGTGAGCGTGCTGACAAGCAAGGT
GCTGGACCTGAAGAACTACATCAACAACAGACTGCTGCCCATCGTGAATCAGCAGAG
CTGCAGAATCAGCAACATCGAGACCGTGATCGAGTTTCAGCAGATGAACAGCAGACT
GCTGGAGATCACAAGAGAGTTCAGCGTGAACGCCGGCGTGACCACCCCCCTGAGCA
CCTACATGCTGACCAACAGCGAACTGCTGAGCCTGATCAACGACATGCCCATCACCA
ACGATCAGAAGAAGCTGATGAGCAGCAACGTGCAGATCGTGAGACAGCAGAGCTAC
 
AGCATCATGAGCATCATCAAGGAAGAGGTGCTGGCCTACGTGGTGCAGCTGCCCATC
TACGGCGTGATCGACACCCCCTGCTGGAAGCTGCACACAAGCCCCCTGTGCACCACC
AATATCAAGGAGGGCAGCAACATCTGCCTGACAAGAACCGACAGAGGCTGGTACTG
CGACAACGCCGGCAGCGTGAGCTTCTTCCCCCAAGCCGACACCTGCAAGGTGCAGA
GCAACAGAGTGTTCTGCGACACCATGAACAGCCTGACCCTGCCTAGCGAGGTGAGCC
TGTGCAACACCGACATCTTCAACAGCAAGTACGACTGCAAGATCATGACAAGCAAGA
CCGACATCAGCAGCAGCGTGATCACAAGCCTGGGCGCCATCGTGAGCTGCTACGGCA
AGACAAAGTGCACAGCTAGCAACAAGAACAGAGGCATCATCAAGACCTTCAGCAAC
GGCTGCGACTACGTGAGCAACAAGGGCGTGGACACCGTGAGCGTGGGCAACACCCT
GTACTACGTGAACAAGCTGGAGGGCAAGAACCTGTACGTGAAGGGCGAGCCCATCAT
CAACTACTACGACCCCCTGGTGTTCCCTAGCGACGAGTTCGACGCTAGCATCAGCCA
AGTGAACGAGAAGATCAATCAGAGCCTGGCCTTCATCAGAAGAAGCGACGAGCTGC
TCAGCGCTATCGGCGGCTACATCCCCGAGGCCCCTAGAGACGGCCAAGCCTACGTGA
GAAAGGACGGCGAGTGGGTGCTCCTGAGCACCTTCCTCTGA
SEQ ID No.25
   RSV-B-PreF-1 (R8) (Del104-145aa) 
MELLIHRSSAIFLTLAVNALYLTSSQNITEEFYQSTCSAVSRGYFSALRTGWYTSVITIE
LSNIKETKCNGTDTKVKLIKQELDKYKNAVTELQLLMQNTPAASAIASGIAVSKVLHLEG
EVNKIKNALLSTNKAVVSLSNGVSVLTSKVLDLKNYINNRLLPIVNQQSCRISNIETVIEF
QQMNSRLLEITREFSVNAGVTTPLSTYMLTNSELLSLINDMPITNDQKKLMSSNVQIVRQ
QSYSIMSIIKEEVLAYVVQLPIYGVIDTPCWKLHTSPLCTTNIKEGSNICLTRTDRGWYCD
NAGSVSFFPQADTCKVQSNRVFCDTMNSLTLPSEVSLCNTDIFNSKYDCKIMTSKTDISSS
VITSLGAIVSCYGKTKCTASNKNRGIIKTFSNGCDYVSNKGVDTVSVGNTLYYVNKLEG
KNLYVKGEPIINYYDPLVFPSDEFDASISQVNEKINQSLAFIRRSDELLHNVNTGKSTTNIM
ITTIIIVIIVVLLSLIAIGLLLYCKAKNTPVTLSKDQLSGINNIAFSK
SEQ ID No.26 encoding polynucleotide of SEQ ID No.25 
ATGGAGCTGCTGATCCACAGAAGCAGCGCCATCTTCCTGACCCTGGCCGTGAAC
GCCCTGTACCTGACAAGCAGCCAAAACATCACCGAGGAGTTCTATCAGAGCACCTGC
AGCGCCGTGAGCAGAGGCTACTTCAGCGCCCTGAGAACCGGCTGGTACACAAGCGT
CATCACCATCGAGCTGAGCAATATTAAGGAGACCAAGTGTAATGGCACCGACACCAA
GGTGAAGCTGATCAAGCAAGAGCTGGACAAGTACAAGAACGCCGTGACCGAGCTGC
 
AGCTGCTGATGCAGAACACCCCTGCCGCTAGCGCCATCGCTAGCGGCATCGCCGTGA
GCAAAGTGCTGCATCTGGAGGGCGAGGTGAACAAGATCAAGAACGCCCTGCTGAGC
ACCAACAAGGCCGTGGTGAGCCTGAGCAACGGCGTGAGCGTGCTGACCTCCAAGGT
GCTGGACCTCAAGAACTACATCAACAACAGACTGCTGCCCATCGTGAATCAGCAGAG
CTGCAGAATCAGCAACATCGAGACCGTGATCGAGTTTCAGCAGATGAACAGCAGACT
GCTGGAGATCACAAGAGAGTTCAGCGTGAACGCCGGCGTGACCACCCCCCTGAGCA
CCTACATGCTGACCAACAGCGAGCTGCTGAGCCTGATCAACGACATGCCCATCACCA
ACGATCAGAAGAAGCTGATGAGCAGCAACGTGCAGATCGTGAGACAGCAGAGCTAC
AGCATCATGAGCATCATCAAGGAAGAGGTGCTGGCCTACGTGGTGCAGCTGCCCATC
TACGGCGTGATCGACACCCCCTGCTGGAAGCTGCACACAAGCCCCCTGTGCACCACC
AACATCAAAGAGGGCTCCAACATCTGCCTGACAAGAACCGACAGAGGCTGGTACTG
CGACAACGCCGGCAGCGTGAGCTTCTTCCCCCAAGCCGACACCTGCAAGGTGCAGA
GCAACAGAGTGTTCTGCGACACCATGAACAGCCTGACCCTGCCTAGCGAGGTGAGCC
TGTGCAACACCGACATCTTCAACAGCAAGTACGACTGCAAGATCATGACAAGCAAGA
CCGACATCAGCAGCAGCGTGATCACAAGCCTGGGCGCCATCGTGAGCTGCTACGGCA
AGACCAAGTGCACCGCTAGCAACAAGAACAGAGGCATCATCAAGACCTTCAGCAAC
GGCTGCGACTACGTGAGCAACAAGGGCGTGGACACCGTGAGCGTGGGCAACACCCT
GTACTACGTGAACAAGCTGGAGGGCAAGAACCTGTACGTGAAGGGCGAGCCCATCAT
CAACTACTACGACCCCCTGGTGTTCCCTAGCGACGAGTTCGACGCTAGCATCAGCCA
AGTGAACGAGAAGATCAATCAGAGCCTGGCCTTCATCAGAAGAAGCGACGAGCTGC
TGCACAACGTGAACACCGGCAAGTCCACCACAAACATCATGATCACCACCATCATTAT
CGTGATCATCGTGGTGCTGCTGTCCCTGATCGCCATCGGCCTGCTCCTGTACTGCAAG
GCCAAGAACACACCTGTGACCCTGAGCAAGGATCAGCTGAGCGGCATCAACAACAT
CGCCTTCAGCAAGTGA
SEQ ID No.27
   RSV-B-PreF-2 (R9) (2 disulfide bond mutations) 
MELLIHRSSAIFLTLAVNALYLTSSQNCTEEFYQSTCSAVSRGYFSALRTGWYTCVITI
ELSNIKETKCNGTDTKVKLIKQELDKYKNAVTELQLLMQNTPAANNRARREAPQYMNY
TINTTKNLNVSISKKRKRRFLGFLLGVGSAIASGIAVSKVLHLEGEVNKIKNALLSTNKAV
VSLSNGVSVCTSKVLDLKNYINNRLLPIVNQQSCRISNIETVIEFQQMNSRLLEITREFSVN
AGVTTPLSTYMLTNSELLSLINDMPITNDQKKLMSSNVQIVRQQSYSIMSIIKEEVLAYVV
 
QLPIYGVIDTPCWKLHTSPLCTTNIKEGSNICLTRTDRGWYCDNAGSVSFFPQADTCKVQ
SNRVFCDTMNSLTLPSEVSLCNTDIFNSKYDCKIMTSKTDISSSVITSLGAIVSCYGKTKCT
ASNKNRGIIKTFSNGCDYVSNKGVDTVSVGNTLYYVNKLECKNLYVKGEPIINYYDPLVF
PSDEFDASISQVNEKINQSLAFIRRSDELLSAIGGYIPEAPRDGQAYVRKDGEWVLLSTFL
SEQ ID No.28 encoding polynucleotide of SEQ ID No.27 
ATGGAGCTGCTGATCCACAGAAGCAGCGCCATCTTCCTGACCCTGGCCGTGAAC
GCCCTGTACCTGACAAGCAGCCAAAACTGCACCGAGGAGTTCTATCAGAGCACCTGC
AGCGCCGTGAGCAGAGGCTACTTCAGCGCCCTGAGAACCGGCTGGTACACCTGCGTC
ATCACAATCGAGCTGAGCAACATCAAAGAGACCAAGTGCAACGGCACCGACACCAA
GGTGAAGCTGATCAAGCAAGAGCTGGACAAGTACAAGAACGCCGTGACCGAGCTGC
AGCTGCTGATGCAGAACACCCCCGCCGCCAACAACAGAGCTAGAAGAGAGGCCCCT
CAGTACATGAACTACACCATCAACACCACCAAGAACCTGAACGTGAGCATCAGCAAG
AAGAGAAAGAGAAGATTCCTGGGCTTCCTGCTGGGCGTGGGCAGCGCCATCGCTAGC
GGCATCGCCGTGTCCAAGGTCCTGCATCTCGAGGGCGAGGTGAACAAGATCAAGAA
CGCCCTGCTGAGCACAAACAAGGCCGTGGTGAGCCTGAGCAACGGCGTGAGCGTGT
GCACAAGCAAGGTGCTGGACCTCAAGAACTACATCAACAACAGACTGCTGCCCATCG
TGAATCAGCAGAGCTGCAGAATCAGCAACATCGAGACCGTGATCGAGTTTCAGCAGA
TGAACAGCAGACTGCTGGAGATCACAAGAGAGTTCAGCGTGAACGCCGGCGTGACC
ACCCCCCTGAGCACCTACATGCTGACCAACAGCGAGCTGCTGAGCCTCATCAACGAC
ATGCCCATCACCAACGATCAGAAGAAGCTGATGAGCAGCAACGTGCAGATCGTGAGA
CAGCAGAGCTACAGCATCATGAGCATCATCAAGGAAGAGGTGCTGGCCTACGTGGTG
CAGCTGCCCATCTACGGCGTGATCGACACCCCCTGCTGGAAGCTGCACACAAGCCCC
CTGTGCACCACCAACATCAAGGAGGGCAGCAACATCTGCCTGACAAGAACCGACAG
AGGCTGGTACTGCGACAACGCCGGCAGCGTGAGCTTCTTCCCCCAAGCCGACACCTG
CAAGGTGCAGAGCAACAGAGTGTTCTGCGACACCATGAACAGCCTGACCCTGCCTA
GCGAGGTGAGCCTGTGCAACACCGACATCTTCAACAGCAAGTACGACTGCAAGATCA
TGACAAGCAAGACCGACATCAGCAGCAGCGTCATCACAAGCCTGGGCGCCATCGTG
AGCTGCTACGGCAAGACCAAATGTACCGCTAGCAACAAGAACAGAGGCATCATCAAG
ACCTTCAGCAACGGCTGCGACTACGTGAGCAACAAGGGCGTGGACACCGTGAGCGT
GGGCAACACCCTGTACTACGTGAACAAGCTGGAGTGCAAGAACCTGTACGTGAAGG
GCGAGCCCATCATCAACTACTACGACCCCCTGGTGTTCCCTAGCGACGAGTTCGACG
 
CTAGCATCAGCCAAGTGAACGAGAAGATCAATCAGAGCCTGGCCTTCATCAGAAGAA
GCGACGAACTGCTGAGCGCCATTGGCGGCTACATCCCCGAGGCCCCTAGAGACGGCC
AAGCCTACGTGAGAAAGGACGGCGAGTGGGTGCTGCTGAGCACCTTCCTGTGA
SEQ ID No.29
   RSV-B-PreF-2 (R10) (2 disulfide bond mutations) 
MELLIHRSSAIFLTLAVNALYLTSSQNCTEEFYQSTCSAVSRGYFSALRTGWYTCVITI
ELSNIKETKCNGTDTKVKLIKQELDKYKNAVTELQLLMQNTPAANNRARREAPQYMNY
TINTTKNLNVSISKKRKRRFLGFLLGVGSAIASGIAVSKVLHLEGEVNKIKNALLSTNKAV
VSLSNGVSVCTSKVLDLKNYINNRLLPIVNQQSCRISNIETVIEFQQMNSRLLEITREFSVN
AGVTTPLSTYMLTNSELLSLINDMPITNDQKKLMSSNVQIVRQQSYSIMSIIKEEVLAYVV
QLPIYGVIDTPCWKLHTSPLCTTNIKEGSNICLTRTDRGWYCDNAGSVSFFPQADTCKVQ
SNRVFCDTMNSLTLPSEVSLCNTDIFNSKYDCKIMTSKTDISSSVITSLGAIVSCYGKTKCT
ASNKNRGIIKTFSNGCDYVSNKGVDTVSVGNTLYYVNKLECKNLYVKGEPIINYYDPLVF
PSDEFDASISQVNEKINQSLAFIRRSDELLHNVNTGKSTTNIMITTIIIVIIVVLLSLIAIGLLL
YCKAKNTPVTLSKDQLSGINNIAFSK
SEQ ID No.30 encoding polynucleotide of SEQ ID No.29 
ATGGAGCTGCTGATCCACAGAAGCAGCGCCATCTTCCTGACCCTGGCCGTGAAC
GCCCTGTACCTGACAAGCAGCCAAAACTGCACCGAGGAGTTCTATCAGAGCACCTGC
AGCGCCGTGAGCAGAGGCTACTTCAGCGCCCTGAGAACCGGCTGGTACACCTGTGTG
ATCACCATCGAGCTGAGCAACATCAAGGAGACCAAATGTAACGGCACCGACACCAA
GGTGAAGCTGATCAAGCAAGAGCTGGACAAGTACAAGAACGCCGTGACCGAGCTGC
AGCTGCTGATGCAGAACACCCCTGCCGCCAACAACAGAGCTAGAAGAGAGGCCCCT
CAGTACATGAACTACACCATCAACACCACCAAGAACCTGAACGTGAGCATCAGCAAG
AAGAGAAAGAGAAGATTCCTGGGCTTCCTGCTGGGCGTGGGCAGCGCCATCGCTAGC
GGCATCGCCGTGAGCAAGGTGCTGCACCTGGAGGGCGAGGTGAACAAGATCAAGAA
CGCCCTGCTGAGCACCAACAAGGCCGTGGTGAGCCTGAGCAACGGCGTGAGCGTGT
GCACAAGCAAAGTGCTGGACCTCAAGAACTACATCAACAACAGACTGCTGCCCATCG
TGAATCAGCAGAGCTGCAGAATCAGCAACATCGAGACCGTGATCGAGTTTCAGCAGA
TGAACAGCAGACTGCTGGAGATCACAAGAGAGTTCAGCGTGAACGCCGGCGTGACC
ACCCCCCTGAGCACCTACATGCTGACCAACAGCGAACTGCTCAGCCTGATTAATGAC
ATGCCCATCACCAACGATCAGAAGAAGCTGATGAGCAGCAACGTGCAGATCGTGAGA
 
CAGCAGAGCTACAGCATCATGAGCATCATCAAGGAAGAGGTGCTGGCCTACGTGGTG
CAGCTGCCCATCTACGGCGTGATCGACACCCCCTGCTGGAAGCTGCACACAAGCCCC
CTGTGCACCACCAACATTAAAGAGGGGAGCAACATCTGCCTGACAAGAACCGACAG
AGGCTGGTACTGCGACAACGCCGGCAGCGTGAGCTTCTTCCCCCAAGCCGACACCTG
CAAGGTGCAGAGCAACAGAGTGTTCTGCGACACCATGAACAGCCTGACCCTGCCTA
GCGAGGTGAGCCTGTGCAACACCGACATCTTCAACAGCAAGTACGACTGCAAGATCA
TGACAAGCAAGACCGACATCAGCAGCAGCGTGATCACAAGCCTCGGCGCCATCGTG
AGCTGCTACGGCAAGACCAAGTGCACCGCTAGCAACAAGAACAGAGGCATCATCAA
GACCTTCAGCAACGGCTGCGACTACGTGAGCAACAAGGGCGTGGACACCGTGAGCG
TGGGCAACACCCTGTACTACGTGAACAAGCTGGAGTGCAAGAACCTGTACGTGAAG
GGCGAGCCCATCATCAACTACTACGACCCCCTGGTGTTCCCTAGCGACGAGTTCGAC
GCTAGCATCAGCCAAGTGAACGAGAAGATCAATCAGAGCCTGGCCTTCATCAGAAGA
AGCGACGAGCTGCTGCACAACGTGAACACCGGCAAGAGCACCACAAATATCATGATC
ACCACCATCATTATCGTGATCATCGTGGTGCTCCTGAGCCTGATCGCCATCGGCCTGCT
CCTGTACTGCAAGGCCAAAAACACCCCCGTGACACTGAGCAAGGATCAGCTGAGCG
GCATCAACAACATCGCCTTCAGCAAGTGA
SEQ ID No.31
   RSV-B-PreF-3 (R11) (Del104-145aa+1 disulfide bond mutation) 
MELLIHRSSAIFLTLAVNALYLTSSQNCTEEFYQSTCSAVSRGYFSALRTGWYTSVITI
ELSNIKETKCNGTDTKVKLIKQELDKYKNAVTELQLLMQNTPAASAIASGIAVSKVLHLE
GEVNKIKNALLSTNKAVVSLSNGVSVLTSKVLDLKNYINNRLLPIVNQQSCRISNIETVIE
FQQMNSRLLEITREFSVNAGVTTPLSTYMLTNSELLSLINDMPITNDQKKLMSSNVQIVR
QQSYSIMSIIKEEVLAYVVQLPIYGVIDTPCWKLHTSPLCTTNIKEGSNICLTRTDRGWYC
DNAGSVSFFPQADTCKVQSNRVFCDTMNSLTLPSEVSLCNTDIFNSKYDCKIMTSKTDIS
SSVITSLGAIVSCYGKTKCTASNKNRGIIKTFSNGCDYVSNKGVDTVSVGNTLYYVNKLE
CKNLYVKGEPIINYYDPLVFPSDEFDASISQVNEKINQSLAFIRRSDELLSAIGGYIPEAPRD
GQAYVRKDGEWVLLSTFL
SEQ ID No.32 encoding polynucleotide of SEQ ID No.31 
ATGGAGCTGCTGATCCACAGAAGCAGCGCCATCTTCCTGACCCTGGCCGTGAAC
GCCCTGTACCTGACAAGCAGCCAAAACTGCACCGAGGAGTTCTATCAGAGCACCTGC
AGCGCCGTGAGCAGAGGCTACTTCAGCGCCCTGAGAACCGGCTGGTACACCTCCGTG
 
ATCACAATCGAACTGAGCAACATTAAGGAGACCAAGTGTAACGGCACCGACACCAA
GGTGAAGCTGATCAAGCAAGAGCTGGACAAGTACAAGAACGCCGTGACCGAGCTGC
AGCTGCTGATGCAGAACACCCCCGCCGCTAGCGCCATCGCTAGCGGCATCGCCGTGT
CCAAAGTGCTGCACCTGGAGGGCGAGGTGAACAAGATCAAGAACGCCCTGCTGAGC
ACAAACAAGGCCGTGGTGAGCCTGAGCAACGGCGTGAGCGTGCTGACAAGCAAGGT
GCTGGACCTGAAGAACTACATCAACAACAGACTGCTGCCCATCGTGAATCAGCAGAG
CTGCAGAATCAGCAACATCGAGACCGTGATCGAGTTTCAGCAGATGAACAGCAGACT
GCTGGAGATCACAAGAGAGTTCAGCGTGAACGCCGGCGTGACCACCCCCCTGAGCA
CCTACATGCTGACCAACAGCGAACTGCTGAGCCTGATCAACGACATGCCCATCACCA
ACGATCAGAAGAAGCTGATGAGCAGCAACGTGCAGATCGTGAGACAGCAGAGCTAC
AGCATCATGAGCATCATCAAGGAAGAGGTGCTGGCCTACGTGGTGCAGCTGCCCATC
TACGGCGTGATCGACACCCCCTGCTGGAAGCTGCACACAAGCCCCCTGTGCACCACC
AATATCAAGGAGGGCAGCAACATCTGCCTGACAAGAACCGACAGAGGCTGGTACTG
CGACAACGCCGGCAGCGTGAGCTTCTTCCCCCAAGCCGACACCTGCAAGGTGCAGA
GCAACAGAGTGTTCTGCGACACCATGAACAGCCTGACCCTGCCTAGCGAGGTGAGCC
TGTGCAACACCGACATCTTCAACAGCAAGTACGACTGCAAGATCATGACAAGCAAGA
CCGACATCAGCAGCAGCGTGATCACAAGCCTGGGCGCCATCGTGAGCTGCTACGGCA
AGACAAAGTGCACAGCTAGCAACAAGAACAGAGGCATCATCAAGACCTTCAGCAAC
GGCTGCGACTACGTGAGCAACAAGGGCGTGGACACCGTGAGCGTGGGCAACACCCT
GTACTACGTGAACAAGCTGGAGTGCAAGAACCTGTACGTGAAGGGCGAGCCCATCAT
CAACTACTACGACCCCCTGGTGTTCCCTAGCGACGAGTTCGACGCTAGCATCAGCCA
AGTGAACGAGAAGATCAATCAGAGCCTGGCCTTCATCAGAAGAAGCGACGAGCTGC
TCAGCGCTATCGGCGGCTACATCCCCGAGGCCCCTAGAGACGGCCAAGCCTACGTGA
GAAAGGACGGCGAGTGGGTGCTCCTGAGCACCTTCCTCTGA
SEQ ID No.33
   RSV-B-PreF-3 (R12) (Del104-145aa+1 disulfide bond mutation) 
MELLIHRSSAIFLTLAVNALYLTSSQNCTEEFYQSTCSAVSRGYFSALRTGWYTSVITI
ELSNIKETKCNGTDTKVKLIKQELDKYKNAVTELQLLMQNTPAASAIASGIAVSKVLHLE
GEVNKIKNALLSTNKAVVSLSNGVSVLTSKVLDLKNYINNRLLPIVNQQSCRISNIETVIE
FQQMNSRLLEITREFSVNAGVTTPLSTYMLTNSELLSLINDMPITNDQKKLMSSNVQIVR
QQSYSIMSIIKEEVLAYVVQLPIYGVIDTPCWKLHTSPLCTTNIKEGSNICLTRTDRGWYC
 
DNAGSVSFFPQADTCKVQSNRVFCDTMNSLTLPSEVSLCNTDIFNSKYDCKIMTSKTDIS
SSVITSLGAIVSCYGKTKCTASNKNRGIIKTFSNGCDYVSNKGVDTVSVGNTLYYVNKLE
CKNLYVKGEPIINYYDPLVFPSDEFDASISQVNEKINQSLAFIRRSDELLHNVNTGKSTTNI
MITTIIIVIIVVLLSLIAIGLLLYCKAKNTPVTLSKDQLSGINNIAFSK
SEQ ID No.34 encoding polynucleotide of SEQ ID No.33

ATGGAGCTGCTGATCCACAGAAGCAGCGCCATCTTCCTGACCCTGGCCGTGAAC
GCCCTGTACCTGACAAGCAGCCAAAACTGCACCGAGGAGTTCTATCAGAGCACCTGC
AGCGCCGTGAGCAGAGGCTACTTCAGCGCCCTGAGAACCGGCTGGTACACAAGCGT
CATCACCATCGAGCTGAGCAATATTAAGGAGACCAAGTGTAATGGCACCGACACCAA
GGTGAAGCTGATCAAGCAAGAGCTGGACAAGTACAAGAACGCCGTGACCGAGCTGC
AGCTGCTGATGCAGAACACCCCTGCCGCTAGCGCCATCGCTAGCGGCATCGCCGTGA
GCAAAGTGCTGCATCTGGAGGGCGAGGTGAACAAGATCAAGAACGCCCTGCTGAGC
ACCAACAAGGCCGTGGTGAGCCTGAGCAACGGCGTGAGCGTGCTGACCTCCAAGGT
GCTGGACCTCAAGAACTACATCAACAACAGACTGCTGCCCATCGTGAATCAGCAGAG
CTGCAGAATCAGCAACATCGAGACCGTGATCGAGTTTCAGCAGATGAACAGCAGACT
GCTGGAGATCACAAGAGAGTTCAGCGTGAACGCCGGCGTGACCACCCCCCTGAGCA
CCTACATGCTGACCAACAGCGAGCTGCTGAGCCTGATCAACGACATGCCCATCACCA
ACGATCAGAAGAAGCTGATGAGCAGCAACGTGCAGATCGTGAGACAGCAGAGCTAC
AGCATCATGAGCATCATCAAGGAAGAGGTGCTGGCCTACGTGGTGCAGCTGCCCATC
TACGGCGTGATCGACACCCCCTGCTGGAAGCTGCACACAAGCCCCCTGTGCACCACC
AACATCAAAGAGGGCTCCAACATCTGCCTGACAAGAACCGACAGAGGCTGGTACTG
CGACAACGCCGGCAGCGTGAGCTTCTTCCCCCAAGCCGACACCTGCAAGGTGCAGA
GCAACAGAGTGTTCTGCGACACCATGAACAGCCTGACCCTGCCTAGCGAGGTGAGCC
TGTGCAACACCGACATCTTCAACAGCAAGTACGACTGCAAGATCATGACAAGCAAGA
CCGACATCAGCAGCAGCGTGATCACAAGCCTGGGCGCCATCGTGAGCTGCTACGGCA
AGACCAAGTGCACCGCTAGCAACAAGAACAGAGGCATCATCAAGACCTTCAGCAAC
GGCTGCGACTACGTGAGCAACAAGGGCGTGGACACCGTGAGCGTGGGCAACACCCT
GTACTACGTGAACAAGCTGGAGTGCAAGAACCTGTACGTGAAGGGCGAGCCCATCAT
CAACTACTACGACCCCCTGGTGTTCCCTAGCGACGAGTTCGACGCTAGCATCAGCCA
AGTGAACGAGAAGATCAATCAGAGCCTGGCCTTCATCAGAAGAAGCGACGAGCTGC
TGCACAACGTGAACACCGGCAAGTCCACCACAAACATCATGATCACCACCATCATTAT

CGTGATCATCGTGGTGCTGCTGTCCCTGATCGCCATCGGCCTGCTCCTGTACTGCAAG
GCCAAGAACACACCTGTGACCCTGAGCAAGGATCAGCTGAGCGGCATCAACAACAT
CGCCTTCAGCAAGTGA

In a second aspect, the present invention provides a composition for preventing and/or treating respiratory syncytial virus infection, where the composition is a compound preparation including a recombinant protein vaccine for preventing and/or treating the respiratory syncytial virus infection and the adenovirus vector vaccine as active components.

The present invention provides a combined drug for preventing and/or treating respiratory syncytial virus infection, and the combined drug includes a recombinant protein vaccine for preventing and/or treating the respiratory syncytial virus infection and the adenovirus vector vaccine, where these vaccines are administered separately or simultaneously.

Further, the recombinant protein vaccine includes the recombinant protein for preventing and/or treating the respiratory syncytial virus infection, and the amino acid sequence of the recombinant protein has more than 70% sequence identity and the same or substantially equivalent biological activity with SEQ ID No.1 or SEQ ID No.2.

Preferably, the amino acid sequence of the recombinant protein is selected from at least one of SEQ ID No.1, SEQ ID No.2, SEQ ID No.3, SEQ ID No.4, SEQ ID No.5 and SEQ ID No.6.

Further, the recombinant protein vaccine is obtained by constructing a protein precursor with an amino acid sequence selected from SEQ ID No.7 or SEQ ID No.8.

Further, a nucleotide sequence encoding the protein precursor is selected from at least one of SEQ ID No.9 and SEQ ID No.10.

Further, a preparation method for a recombinant protein in the recombinant protein vaccine includes the following steps: culturing a host cell to express the protein or the protein precursor, and then recovering the protein.

Further, an insect cell or a mammalian cell is used as the host cell. Preferably, the insect cell is selected from at least one of a sf9 cell, a sf21 cell and a Hi5 cell. Preferably, the mammalian cell is a CHO cell. Further preferably, the CHO cell expression vector is pTTS or FTP-002.

The host cell includes a recombinant vector, and the recombinant vector includes nucleotides encoding the protein or the protein precursor. Further, the recombinant vector is an insect baculovirus expression vector or a mammalian cell expression vector. Preferably, the insect baculovirus expression vector is pFastBac1. Preferably, the mammalian cell expression vector is a CHO cell expression vector.

Further, the recombinant protein vaccine includes the recombinant protein and/or the protein precursor, and pharmaceutically acceptable excipients or adjuvant components.

Further, the adjuvant component is an immunologic adjuvant. Preferably, the immunologic adjuvant is selected from at least one of the following: aluminum salt, calcium salt, phytosaponin, phytopolysaccharide, monophosphate lipid A, muramyl dipeptide, muramyl tripeptide, squalene-based oil-in-water emulsion, recombinant cholera toxin, GM-CSF cytokine, lipid and cationic liposome material, and CpG ODN.

Further, the squalene-based oil-in-water emulsion is MF59. Further, the aluminum salt is selected from at least one of aluminum hydroxide and alum. Further, the calcium salt is tricalcium phosphate. Further, the phytosaponin is either QS-21 or ISCOM. Further, the phytopolysaccharide is astragalus polysaccharide. Further, the lipid is selected from at least one of the following: phosphatidylethanolamine (PE), phosphatidylcholine (PC), cholesterol (Chol), and dioleoylphosphatidylethanolamine (DOPE). Further, the cationic liposome material is selected from at least one of the following: (2,3-dioleoyloxypropyl) trimethyl ammonium chloride (DOTAP), N-[1-(2,3-dioleoyl chloride) propyl]-N,N,N-trimethylamine chloride (DOTMA), cationic cholesterol (DC-Chol), dimethyl-2,3-dioleyloxypropyl-2-(2-spermidinecarboxamido)ethyl ammonium trifluoroacetate (DOSPA), trimethyldodecylammonium bromide (DTAB), trimethyltetradecylammonium bromide (TTAB), trimethylhexadecylammonium bromide (CTAB), and dimethyldioctadecylammonium bromide (DDAB).

Further, the composition or the combined drug is formulated as an intradermal injection preparation or a subcutaneous injection preparation, an intramuscular injection preparation, an intravenous injection preparation, an oral preparation or a nasal spray preparation; and preferably, the vaccine is formulated as the intramuscular injection preparation and the nasal spray preparation.

In a third aspect, the present invention provides use of the adenovirus vector vaccine, the composition, or the combined drug in preparing drugs for preventing and/or treating respiratory syncytial virus infection.
SEQ ID No.1 is RSV subtype A F sequence: QNITEEFYQSTCSAVSKGYLSALRTGWYTSVITIELSNIKENKCNGTDAKVKLIKQEL 
DKYKNAVTELQLLMQSTPPTNNRARRELPRFMNYTLNNAKKTNVTLSKKRKRRFLGFL
LGVGSAIASGVAVSKVLHLEGEVNKIKSALLSTNKAVVSLSNGVSVLTSKVLDLKNYIDK
QLLPIVNKQSCSISNIETVIEFQQKNNRLLEITREFSVNAGVTTPVSTYMLTNSELLSLIND
MPITNDQKKLMSNNVQIVRQQSYSIMSIIKEEVLAYVVQLPLYGVIDTPCWKLHTSPLCT
TNTKEGSNICLTRTDRGWYCDNAGSVSFFPQAETCKVQSNRVFCDTMNSLTLPSEINLCN
VDIFNPKYDCKIMTSKTDVSSSVITSLGAIVSCYGKTKCTASNKNRGIIKTFSNGCDYVSN
KGMDTVSVGNTLYYVNKQEGKSLYVKGEPIINFYDPLVFPSDEFDASISQVNEKINQSLA
FIRKSDELLHNVNAGKSTTNIMITTIIIVIIVILLSLIAVGLLLYCKARSTPVTLSKDQLSGIN
NIAFSN
SEQ ID No.2 is RSV subtype B F sequence: 
QNITEEFYQSTCSAVSRGYFSALRTGWYTSVITIELSNIKETKCNGTDTKVKLIKQEL
DKYKNAVTELQLLMQNTPAANNRARREAPQYMNYTINTTKNLNVSISKKRKRRFLGFL
LGVGSAIASGIAVSKVLHLEGEVNKIKNALLSTNKAVVSLSNGVSVLTSKVLDLKNYINN
RLLPIVNQQSCRISNIETVIEFQQMNSRLLEITREFSVNAGVTTPLSTYMLTNSELLSLIND
MPITNDQKKLMSSNVQIVRQQSYSIMSIIKEEVLAYVVQLPIYGVIDTPCWKLHTSPLCTT
NIKEGSNICLTRTDRGWYCDNAGSVSFFPQADTCKVQSNRVFCDTMNSLTLPSEVSLCNT
DIFNSKYDCKIMTSKTDISSSVITSLGAIVSCYGKTKCTASNKNRGIIKTFSNGCDYVSNK
GVDTVSVGNTLYYVNKLEGKNLYVKGEPIINYYDPLVFPSDEFDASISQVNEKINQSLAFI
RRSDELLHNVNTGKSTTNIMITTIIIVIIVVLLSLIAIGLLLYCKAKNTPVTLSKDQLSGINNI
AFSK
SEQ ID No.3: optimized RSV subtype A F protein truncated sequence 
QNCTEEFYQSTCSAVSKGYLSALRTGWYTCVITIELSNIKENKCNGTDAKVKLIKQE
LDKYKNAVTELQLLMQSTPPTSAIASGVAVSKVLHLEGEVNKIKSALLSTNKAVVSLSNG
VSVCTSKVLDLKNYIDKQLLPIVNKQSCSISNIETVIEFQQKNNRLLEITREFSVNAGVTTP
VSTYMLTNSELLSLINDMPITNDQKKLMSNNVQIVRQQSYSIMSIIKEEVLAYVVQLPLY
GVIDTPCWKLHTSPLCTTNTKEGSNICLTRTDRGWYCDNAGSVSFFPQAETCKVQSNRV
FCDTMNSLTLPSEINLCNVDIFNPKYDCKIMTSKTDVSSSVITSLGAIVSCYGKTKCTASN
KNRGIIKTFSNGCDYVSNKGMDTVSVGNTLYYVNKQECKSLYVKGEPIINFYDPLVFPSD
EFDASISQVNEKINQSLAFIRKSDELL
SEQ ID No.4: optimized RSV subtype B F protein truncated sequence QNCTEEFYQSTCSAVSRGYFSALRTGWYTCVITIELSNIKETKCNGTDTKVKLIKQEL 
DKYKNAVTELQLLMQNTPAASAIASGIAVSKVLHLEGEVNKIKNALLSTNKAVVSLSNG
VSVCTSKVLDLKNYINNRLLPIVNQQSCRISNIETVIEFQQMNSRLLEITREFSVNAGVTTP
LSTYMLTNSELLSLINDMPITNDQKKLMSSNVQIVRQQSYSIMSIIKEEVLAYVVQLPIYG
VIDTPCWKLHTSPLCTTNIKEGSNICLTRTDRGWYCDNAGSVSFFPQADTCKVQSNRVFC
DTMNSLTLPSEVSLCNTDIFNSKYDCKIMTSKTDISSSVITSLGAIVSCYGKTKCTASNKN
RGIIKTFSNGCDYVSNKGVDTVSVGNTLYYVNKLECKNLYVKGEPIINYYDPLVFPSDEF
DASISQVNEKINQSLAFIRRSDELL
SEQ ID No.5: SAIG and foldon sequences added to optimized RSV subtype A F truncated sequence 
QNCTEEFYQSTCSAVSKGYLSALRTGWYTCVITIELSNIKENKCNGTDAKVKLIKQELD
KYKNAVTELQLLMQSTPPTSAIASGVAVSKVLHLEGEVNKIKSALLSTNKAVVSLSNGVS
VCTSKVLDLKNYIDKQLLPIVNKQSCSISNIETVIEFQQKNNRLLEITREFSVNAGVTTPVS
TYMLTNSELLSLINDMPITNDQKKLMSNNVQIVRQQSYSIMSIIKEEVLAYVVQLPLYGVI
DTPCWKLHTSPLCTTNTKEGSNICLTRTDRGWYCDNAGSVSFFPQAETCKVQSNRVFCD
TMNSLTLPSEINLCNVDIFNPKYDCKIMTSKTDVSSSVITSLGAIVSCYGKTKCTASNKNR
GIIKTFSNGCDYVSNKGMDTVSVGNTLYYVNKQECKSLYVKGEPIINFYDPLVFPSDEFD
ASISQVNEKINQSLAFIRKSDELLSAIGGYIPEAPRDGQAYVRKDGEWVLLSTFL
SEQ ID No.6: SAIG and foldon sequences added to optimized RSV subtype B F truncated sequence 
QNCTEEFYQSTCSAVSRGYFSALRTGWYTCVITIELSNIKETKCNGTDTKVKLIKQEL
DKYKNAVTELQLLMQNTPAASAIASGIAVSKVLHLEGEVNKIKNALLSTNKAVVSLSNG
VSVCTSKVLDLKNYINNRLLPIVNQQSCRISNIETVIEFQQMNSRLLEITREFSVNAGVTTP
LSTYMLTNSELLSLINDMPITNDQKKLMSSNVQIVRQQSYSIMSIIKEEVLAYVVQLPIYG
VIDTPCWKLHTSPLCTTNIKEGSNICLTRTDRGWYCDNAGSVSFFPQADTCKVQSNRVFC
DTMNSLTLPSEVSLCNTDIFNSKYDCKIMTSKTDISSSVITSLGAIVSCYGKTKCTASNKN
RGIIKTFSNGCDYVSNKGVDTVSVGNTLYYVNKLECKNLYVKGEPIINYYDPLVFPSDEF
DASISQVNEKINQSLAFIRRSDELLSAIGGYIPEAPRDGQAYVRKDGEWVLLSTFL
SEQ ID No.7 signal peptide + optimized RSV subtype A F protein truncated sequence+ (SAIG) + foldon sequence + thrombin cleavage site + His 6 tag + strep II tag 
MELLILKANAITTILTAVTFCFASGQNCTEEFYQSTCSAVSKGYLSALRTGWYTCVITI
ELSNIKENKCNGTDAKVKLIKQELDKYKNAVTELQLLMQSTPPTSAIASGVAVSKVLHLE
 
GEVNKIKSALLSTNKAVVSLSNGVSVCTSKVLDLKNYIDKQLLPIVNKQSCSISNIETVIEF
QQKNNRLLEITREFSVNAGVTTPVSTYMLTNSELLSLINDMPITNDQKKLMSNNVQIVRQ
QSYSIMSIIKEEVLAYVVQLPLYGVIDTPCWKLHTSPLCTTNTKEGSNICLTRTDRGWYCD
NAGSVSFFPQAETCKVQSNRVFCDTMNSLTLPSEINLCNVDIFNPKYDCKIMTSKTDVSS
SVITSLGAIVSCYGKTKCTASNKNRGIIKTFSNGCDYVSNKGMDTVSVGNTLYYVNKQE
CKSLYVKGEPIINFYDPLVFPSDEFDASISQVNEKINQSLAFIRKSDELLSAIGGYIPEAPRD
GQAYVRKDGEWVLLSTFLVPRGSHHHHHHGSWSHPQFEK
SEQ ID No.8 signal peptide + constructed RSV subtype B F protein truncated sequence + (SAIG) + foldon sequence + thrombin cleavage site + His 6 tag + strep II tag 
MELLILKANAITTILTAVTFCFASGQNCTEEFYQSTCSAVSRGYFSALRTGWYTCVITI
ELSNIKETKCNGTDTKVKLIKQELDKYKNAVTELQLLMQNTPAASAIASGIAVSKVLHLE
GEVNKIKNALLSTNKAVVSLSNGVSVCTSKVLDLKNYINNRLLPIVNQQSCRISNIETVIE
FQQMNSRLLEITREFSVNAGVTTPLSTYMLTNSELLSLINDMPITNDQKKLMSSNVQIVR
QQSYSIMSIIKEEVLAYVVQLPIYGVIDTPCWKLHTSPLCTTNIKEGSNICLTRTDRGWYC
DNAGSVSFFPQADTCKVQSNRVFCDTMNSLTLPSEVSLCNTDIFNSKYDCKIMTSKTDIS
SSVITSLGAIVSCYGKTKCTASNKNRGIIKTFSNGCDYVSNKGVDTVSVGNTLYYVNKLE
CKNLYVKGEPIINYYDPLVFPSDEFDASISQVNEKINQSLAFIRRSDELLSAIGGYIPEAPRD
GQAYVRKDGEWVLLSTFLVPRGSHHHHHHGSWSHPQFEK
SEQ ID No.9 encoding a polynucleotide sequence shown in SEQ ID No.7, which is codon-optimized based on an optimized RSV subtype A F protein 
ATGGAGCTGCTGATCCTGAAGGCCAACGCCATCACAACCATCCTGACAGCCGTG
ACATTCTGCTTCGCCTCCGGCCAGAATTGCACCGAGGAGTTCTACCAGTCCACATGTT
CCGCCGTGAGCAAGGGCTACCTGTCCGCCCTGAGAACCGGCTGGTACACCTGTGTGA
TCACCATCGAGCTGTCCAATATCAAGGAGAACAAGTGCAACGGCACCGACGCCAAG
GTGAAGCTGATCAAGCAGGAGCTGGACAAGTACAAGAACGCCGTGACCGAGCTGCA
GCTGCTGATGCAGAGCACACCTCCCACCTCCGCCATCGCCAGCGGCGTTGCTGTGTC
CAAGGTGCTGCACCTGGAGGGCGAGGTGAACAAGATCAAGTCCGCCCTGCTGTCCA
CCAACAAGGCCGTGGTGTCCCTGAGCAACGGCGTGTCCGTGTGCACCAGCAAGGTG
CTGGATCTGAAGAATTACATCGACAAGCAGCTGCTGCCCATCGTGAATAAGCAGAGC
TGTAGCATCAGCAACATCGAGACCGTGATCGAGTTCCAGCAGAAGAATAACAGGCTG
CTGGAGATCACCAGGGAGTTCTCCGTGAACGCCGGCGTGACCACACCCGTGAGCAC
 
CTACATGCTGACCAATTCCGAGCTGCTGTCCCTGATCAATGATATGCCCATCACCAATG
ATCAGAAGAAGCTGATGTCCAACAACGTGCAGATCGTGAGGCAGCAGTCCTACAGCA
TCATGTCCATCATCAAGGAGGAGGTGCTGGCCTACGTGGTGCAGCTGCCTCTGTACG
GCGTGATCGATACCCCTTGCTGGAAGCTGCACACCAGCCCCCTGTGCACAACAAACA
CCAAGGAGGGCAGCAACATCTGCCTGACAAGGACAGACAGAGGCTGGTACTGTGAT
AACGCCGGCAGCGTGTCCTTCTTCCCCCAGGCCGAGACCTGCAAGGTGCAGTCCAAC
AGAGTGTTCTGCGATACCATGAACTCCCTGACCCTGCCTAGCGAGATCAACCTGTGCA
ACGTGGATATCTTCAATCCTAAGTACGATTGTAAGATCATGACCTCCAAGACAGACGT
GTCCAGCAGCGTGATCACATCCCTGGGCGCCATCGTGTCCTGTTACGGCAAGACCAA
GTGTACCGCCTCCAATAAGAATAGGGGCATCATCAAGACATTCTCCAATGGCTGCGAT
TACGTGTCCAACAAGGGCATGGATACCGTGAGCGTGGGCAATACACTGTACTACGTG
AACAAGCAGGAGTGTAAGTCCCTGTACGTGAAGGGCGAGCCTATCATCAACTTCTAC
GACCCCCTGGTGTTCCCTAGCGACGAGTTCGACGCCAGCATCTCCCAGGTGAACGAG
AAGATCAACCAGTCCCTGGCCTTCATCAGGAAGAGCGACGAGCTGCTGAGCGCCATC
GGCGGCTACATCCCTGAGGCCCCTAGGGACGGCCAGGCCTACGTGAGGAAGGACGG
CGAGTGGGTGCTGCTGTCCACATTCCTGGTGCCTAGAGGCTCCCACCACCACCACCA
TCACGGCAGCTGGTCCCACCCTCAGTTCGAGAAGTGA
SEQ ID No.10 encoding a polynucleotide sequence shown in SEQ ID No.8, which is codon-optimized based on an optimized RSV subtype B F protein

ATGGAGCTGCTGATCCACAGGAGCAGCGCCATCTTCCTGACCCTGGCCGTGAAC
GCCCTGTACCTGACAAGCTCCCAGAACTGCACCGAGGAGTTCTACCAGAGCACCTGT
AGCGCCGTGTCCAGGGGCTACTTCAGCGCCCTGAGGACCGGCTGGTACACCTGCGTG
ATCACCATCGAGCTGTCCAATATCAAGGAGACCAAGTGTAACGGCACCGATACCAAG
GTGAAGCTGATCAAGCAGGAGCTGGATAAGTACAAGAATGCCGTGACCGAGCTGCA
GCTGCTGATGCAGAATACACCTGCCGCCTCCGCCATCGCCAGCGGAATCGCTGTGTCC
AAGGTGCTGCACCTGGAGGGCGAGGTGAATAAGATCAAGAATGCCCTGCTGTCCACA
AACAAGGCCGTGGTGAGCCTGAGCAACGGCGTGAGCGTGTGTACATCCAAGGTGCT
CGACCTGAAGAACTACATCAACAATAGACTGCTGCCTATCGTGAACCAGCAGAGCTG
TAGGATCAGCAATATCGAGACAGTGATCGAGTTCCAGCAGATGAACAGCAGGCTGCT
GGAGATCACAAGAGAGTTCTCCGTGAATGCCGGCGTGACAACACCTCTGTCCACCTA
CATGCTGACAAATTCCGAGCTGCTGTCCCTGATCAATGATATGCCTATCACAAACGATC

AGAAGAAGCTGATGAGCTCCAATGTGCAGATCGTGAGGCAGCAGAGCTACTCCATCA
TGTCCATCATCAAGGAGGAGGTGCTGGCCTACGTGGTGCAGCTGCCCATCTACGGCG
TGATCGACACCCCCTGCTGGAAGCTGCACACATCCCCTCTGTGCACCACCAATATCAA
GGAAGGCTCCAATATCTGTCTGACCAGGACCGATAGGGGCTGGTACTGCGATAATGCC
GGCTCCGTGAGCTTCTTCCCTCAGGCCGACACCTGTAAGGTGCAGAGCAATAGAGTG
TTCTGTGATACCATGAACTCCCTGACCCTGCCCTCCGAGGTGAGCCTGTGTAATACAG
ATATCTTCAATAGCAAGTACGATTGCAAGATCATGACCAGCAAGACCGATATCTCCAG
CAGCGTGATCACCTCCCTGGGCGCCATCGTGAGCTGCTACGGCAAGACCAAGTGTAC
CGCCTCCAACAAGAACAGAGGCATCATCAAGACCTTCAGCAATGGCTGTGACTACGT
GAGCAACAAGGGCGTGGACACCGTGAGCGTGGGCAATACACTGTACTACGTGAACA
AGCTGGAGTGTAAGAACCTGTACGTGAAGGGCGAGCCTATCATCAATTACTACGACC
CTCTGGTGTTCCCCTCCGATGAGTTCGACGCCAGCATCTCCCAGGTGAACGAGAAGA
TCAATCAGTCCCTGGCCTTCATCAGAAGATCCGATGAGCTGCTGAGCGCCATCGGCG
GCTACATCCCCGAGGCCCCTAGAGATGGCCAGGCCTACGTGAGAAAGGATGGCGAGT
GGGTGCTGCTGTCCACCTTCCTGGTGCCCAGGGGCAGCCACCACCACCACCATCACG
GCTCCTGGTCCCACCCCCAGTTCGAGAAGTGA.

Beneficial effects: in the present invention, a series of adenovirus vector vaccines against respiratory syncytial virus infection is designed based on the optimized F protein sequences of the respiratory syncytial virus. Animal studies demonstrate that a recombinant adenovirus vector vaccine carrying the codon-optimized subtype A RSV-PreF (RSV-A-PreF) gene can simultaneously induce the production of serum antibodies against pre-fusion (pre-F) and post-fusion (post-F) glycoproteins after animals are immunized. In addition, such a vaccine can induce the production of high-efficacy antibodies against the pre-fusion (pre-F) and post-fusion (post-F) glycoproteins in the bronchus. Notably, the adenovirus vector vaccine has potent humoral immunoprotective efficacy against RSV.

The recombinant adenovirus vaccine including the optimized subtype A RSV-PreF (RSV-A-PreF) R2 gene (the amino acid sequence is shown in SEQ ID No.13) exhibits potent humoral immunoprotective efficacy and safety after the animals are immunized, and can be prepared on a large scale to help hosts to resist respiratory syncytial virus infection, which has a broad application prospect.

### BRIEF DESCRIPTION OF THE DRAWINGS

FIG. 1 shows a plaque diagram of an RSV recombinant adenovirus in Embodiment 1;
FIG. 2 shows a PCR identification diagram of the RSV recombinant adenovirus in Embodiment 1:1 negative control, 2 Ad-RSV-PreF;
FIG. 3 shows a result diagram of anti-preF IgG in sera of mice intranasally immunized with different doses of adenovirus vector RSV vaccine R1 in Embodiment 3;
FIG. 4 shows a result diagram of anti-preF IgG in sera of mice intranasally immunized with different doses of adenovirus vector RSV vaccine R2 in Embodiment 3;
FIG. 5 shows a result diagram of anti-preF IgG in sera of mice intranasally immunized with different doses of adenovirus vector RSV vaccine R3 in Embodiment 3;
FIG. 6 shows a result diagram of anti-preF IgG in sera of mice intranasally immunized with different doses of adenovirus vector RSV vaccine R4 in Embodiment 3;
FIG. 7 shows a result diagram of anti-preF IgG in sera of mice intranasally immunized with different doses of adenovirus vector RSV vaccine R5 in Embodiment 3;
FIG. 8 shows a result diagram of anti-postF IgG in sera of mice intranasally immunized with different doses of adenovirus vector RSV vaccine R2 in Embodiment 3;
FIG. 9 shows a result diagram of anti-preF IgG in sera of mice intramuscularly immunized with different doses of adenovirus vector RSV vaccine R2 in Embodiment 3;
FIG. 10 shows a result diagram of anti-postF IgG in sera of mice intramuscularly immunized with different doses of adenovirus vector RSV vaccine R2 in Embodiment 3;
FIG. 11 shows a result diagram of anti-postF IgG in bronchi of mice intranasally immunized with different doses of adenovirus vector RSV vaccine R2 in Embodiment 3;
FIG. 12 shows a result diagram of anti-preF IgG in bronchi of mice intranasally immunized with different doses of adenovirus vector RSV vaccine R2 in Embodiment 3;
FIG. 13 shows a result diagram of anti-postF IgA in bronchi of mice intranasally immunized with different doses of adenovirus vector RSV vaccine R2 in Embodiment 3;
FIG. 14 shows a result diagram of anti-preF in bronchi of mice intranasally immunized with different doses of adenovirus vector RSV vaccine R2in Embodiment 3;
FIG. 15 shows a result diagram of anti-postF IgG in sera of rats intranasally immunized with different doses of adenovirus vector RSV vaccine R2 in Embodiment 3;
FIG. 16 shows a result diagram of anti-preF IgG in sera of rats intranasally immunized with different doses of adenovirus vector RSV vaccine R2in Embodiment 3;
FIG. 17 shows a result diagram of RSV neutralizing antibodies in sera of mice intranasally immunized with an adenovirus vector RSV vaccine R2 in Embodiment 4;
FIG. 18 shows a result diagram of virus load in challenge experiment lung tissues of cotton rats intranasally immunized with an adenovirus vector RSV vaccine R2 in Embodiment 5; and
FIG. 19 shows a pathological result diagram of challenge experiment lung tissues of cotton rats intranasally immunized with an adenovirus vector RSV vaccine R2 in Embodiment 5.

### DESCRIPTION OF THE EMBODIMENTS

The solution of the present invention will be explained with reference to specific embodiments. It will be understood by those skilled in the art that the following embodiments are merely intended for illustrating the present invention, instead of limiting the scope of the present invention. If specific technologies or conditions are not specified in the embodiments, technologies or conditions described in literatures in the art or product specifications should be followed. If manufacturers are not indicated with respect to reagents or instruments used, they are all conventional products that can be purchased from the market.

### Embodiment 1 Preparation of adenovirus vector RSV vaccine

### (1) Construction of RSV recombinant adenovirus

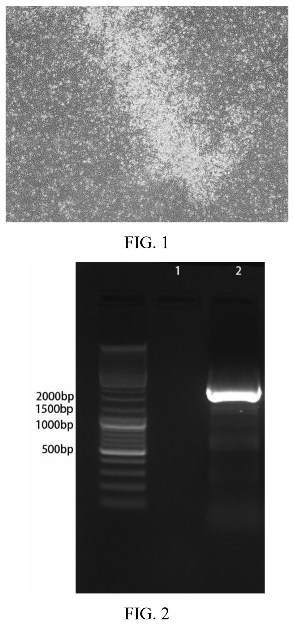

1. Amino acid truncation or amino acid mutation was performed based on the F protein sequence of subtype A RSV to obtain a series of PreF candidate sequences. Suzhou Jinweizhi Biotechnology Co., Ltd. was entrusted to optimize human-derived codons and synthesize genes to obtain the optimized subtype A RSV-PreF (RSV-A-PreF) gene (with amino acid sequences shown in SEQ ID No.11, SEQ ID No.13, SEQ ID No.15, SEQ ID No.17 and SEQ ID No.19 respectively; and with corresponding nucleotide sequences encoding the amino acid sequences shown in SEQ ID No.12, SEQ ID No.14, SEQ ID No.16, SEQ ID No.18 and SEQ ID No.20 respectively).
2. The optimized subtype A RSV-PreF (RSV-A-PreF) gene was inserted between XmaI and SacI restriction enzyme cutting sites of pDC516 vector by seamless cloning (homologous recombination), and the obtained shuttle plasmid was named pDC516-RSV-A-PreF. According to the instructions of lipofectamine 3000 transfection kit, the shuttle plasmid pDC516-RSV-A-PreF (2 ug) including RSV-A-PreF gene and the backbone plasmid pBHGfrt_E1, 3FLP (4 ug) of AdMax adenovirus system were co-transfected into HEK293A cells to package the recombinant adenovirus. A specific process was as follows:
   1) HEK293A cells with 8×10⁵ cells/well were inoculated into a six-well plate, cultured in high-glucose DMEM+10% FBS culture medium, and placed in a cell incubator with 5% CO2 at 37°C for overnight.
   2) the next day, the high-glucose DMEM+ 2% FBS was used for culture medium exchange, and the backbone plasmid (pBHGfrt_E1, 3FLP) and the shuttle plasmid (pDC516-RSV-A-PreF) were co-transfected into HEK293A cells with lipofectamine 3000. The specific steps were as follows: 4 µg of backbone plasmid and 2 µg of shuttle plasmid were taken for each transfection well and diluted with 125 µL of Opti-MEM culture medium, and then 12 µL of P3000 reagent was added into the diluent; another 1.5 mL EP tube was taken and 7.5 µL lipofectamine 3000 was diluted with 125 µL Opti-MEM culture medium; the diluted plasmid and the diluted lipofectamine 3000 were mixed at a ratio of 1: 1, the resulting mixture was incubated at room temperature for 10-15 minutes and then added into the cells, and the cells continued to be cultured; after the cells were fully grown, they were passed on to a 25 cm² cell culture bottle, and the signs of cell cytotoxicity were observed every day; then, when the cells were fully grown at the bottom of the bottle, they were passed on to a 75 cm² cell culture bottle until the cells appeared distinct plaque, as shown in FIG. 1, and the viral stock was harvested when most of the cells became lysed and fell off from the growth surface.
   3) The cytotoxic cell culture was harvested and centrifuged at 1200 rpm for 3 minutes, the virus-containing supernatant was aspirated, the cell pellets were resuspended with a 1/10 culture volume of the virus-containing supernatant, and subjected to three cycles of freezing and thawing in a -80°C refrigerator and a 37°C water bath. Then, the prepared product was centrifuged at 3000 rpm for 20 minutes, the virus-containing supernatant was harvested and mixed with the above virus-containing supernatant, and the resulting mixture is the virus strains of the adenovirus vaccine.
3. Identification of virus strains
   50 µL of vaccine candidate virus strain stock was taken and added with 2 µL of protease K, and the resulting mixture was digested at 50°C for 30 minutes to release the virus genome; the resulting digest was used as a template for PCR amplification of the gene sequence, and the PCR product would be sequenced and identified after its electrophoresis gel was recovered. As shown in FIG. 2, 1 represents negative control, and 2 represents Ad5 vector encoding preF RSV F protein (Ad-RSV-PreF) with stable conformation. The conditions, parameters and primers for PCR amplification are as follows:
   The conditions of PCR amplification are as follows: denaturation: 98°C, 2 minutes, denaturation: 98°C, 10 seconds, annealing: 60°C, 15 seconds, extension: 72°C, 1 minute, extension: 72°C, 5 minutes, number of cycles: 35.

The primers for PCR amplification are as follows:
Ad2304-F: ACGTGGGTATAAGAGGCGCGAC (SEQ ID No.35)
Ad2304-R: CTTCGGATCTTCGATGCTAGACGATCC (SEQ ID No.36)

### (2) Large-scale amplification of RSV recombinant adenovirus

Qualified RSV recombinant adenovirus strains were amplified step by step in 293 cells. A specific process was as follows: 1.0-4.0×10⁶/mL 293H cells were added according to MOI=1-30, and a virus culture was harvested when the cell vitality was 50%-80%, and a master virus seed stock and a working virus seed stock were prepared. The recombinant adenovirus vaccine was amplified in a cell factory or a bioreactor, and the virus culture was harvested after most of the cells became lysed. The cells and viruses were amplified in the bioreactor by the following steps: adding PBS into the bioreactor and sterilizing the bioreactor; after sterilization, discarding PBS from the bioreactor; adding a cell culture fluid into the bioreactor; when the operating conditions were kept at 37°C, pH 7.0, DO 50% and 50 rpm, harvesting 293H cells that were in a medicine bottle and in a logarithmic growth period and inoculating these cells into the bioreactor, where the inoculated cells had a density of 0.5-1.0×10⁶ cells/mL; supplementing the cell culture fluid to 5 L, where the cell culture conditions of the bioreactor were as follows: temperature of 37°C, rotation speed of 30-50 rpm, pH 7.15-7.25, and DO 30%-50%; taking a culture sample every day to detect the glucose concentration, cell density and cell morphology on a microcarrier; when the density of the cell in the bioreactor reached 1.0-4.0×10⁶ cells/mL, inoculating the recombinant adenovirus strains in the bioreactor, with MOI of 1-30; after inoculation, taking an inoculation sample every day to detect the glucose concentration, and virus titer in a culture supernatant and cell pellet, and observing the morphology of cells on the microcarrier; when most of the cells fell off the microcarrier, stopping the culture, adding a virus lysis solution to the bioreactor, with a final concentration of 0.05%-1% Tween 80 at 37°C for 2-4 hours, and then harvesting a virus solution.

### (3) Purification of RSV recombinant adenovirus

The harvested virus was purified by cesium chloride ultracentrifugation or ion exchange chromatography, and the purified adenovirus was directly aliquoted and stored at -20°C in the dark. A specific process was as follows:

### (1) Purification of adenovirus by cesium chloride ultracentrifugation

The harvested virus culture was centrifuged at 1200 g for 10 minutes, and the virus-containing culture supernatant was aspirated. The cell pellet was resuspended with a 1/10 culture volume of the virus-containing supernatant, and was subjected to three cycles of freezing and thawing in a -80°C refrigerator and a 37°C water bath, and centrifuged at 3000 rpm for 10-20 minutes, and the supernatant was aspirated. The virus-containing culture supernatant was concentrated 10 folds with a 100K-300K ultrafiltration membrane; 1.4 g/mL cesium chloride solution (53 g cesium chloride + 87 mL 10 mM Tris-HCl, pH 7.9) and 1.2 g/mL cesium chloride solution (26.8 g cesium chloride + 92 mL 10 mM Tris-HCl, pH 7.9) were prepared; 8 mL 1.4 g/mL cesium chloride solution and 6 mL 1.2 g/mL cesium chloride solution were slowly added into an overspeed tube in sequence, and finally 20 mL of the virus-containing culture supernatant was added at the top of discontinuous gradient, and the balanced tube was centrifuged at 100000×g for 90 minutes at 4°C; after centrifugation, a blue virus band was aspirated by a syringe, followed by dialysis to remove cesium chloride, and the purified virus was stored at -80°C.

### (2) Purification of adenovirus by ion exchange chromatography

The virus culture was harvested and lysed with0.05%-1% Tween 80 at 37°C for 2-4 hours; the lysed culture was filtrated with 1.2 µm and 0.45 µm cartridge filters; the culture sample was concentrated 10 folds with a tangential flow membrane with a molecular weight of 100-300 kD, and washed with 10 volumes of a wash buffer (50 mM Tris-HCl, 2 mM MgCl₂, 0-500 mM NaCl, pH 8.0), and then harvested; nuclease was added to the washed sample, with a final concentration of 10-50 U/mL; the mixture was digested at 37°C for 1-3 hours; the washed sample was subjected to anion exchange chromatography with Q Sepharose XL, Source 30Q, Source 15Q or other fillers, and the specific process was as follows: the buffer was equilibrated at 20 mL/minute and five column volumes; after equilibration, the sample was loaded at 10 mL/minute; after loading, the buffer was equilibrated to a conductivity level; the sample was eluted by linear gradient under the elution conditions of 100% low-salt buffer and 100% high-salt buffer, the elution column volume of 10 V, and the flow rate of 10 mL/minute; and each elution peak was harvested; after elution, the column was regenerated with 2M NaCl buffer for 5-10 column volumes at a speed of 20 mL/minute. The virus peak was harvested, and then the eluted virus sample was dialyzed or filtered by tangential flow for buffer replacement.

The RSV recombinant adenovirus prepared by the above method was used as a vector vaccine for subsequent research such as animal immunity.

### Embodiment 2 Animal preparation, animal immunization and sample harvesting

1. Immunization procedure of intramuscular immunization adenovirus vector vaccine: female BALB/c mice aged 6-8 weeks were purchased from Charles River Laboratories, and the mice were housed under specific pathogen-free (SPF) conditions in National Key Laboratory of Biotherapy, Sichuan University. Mice were intramuscularly immunized twice on days 0 and 21, and the immunizing doses of used adenovirus vector RSV vaccines R1, R2, R3, R4 and R5 (with amino acid sequences shown in SEQ ID No.11, SEQ ID No.13, SEQ ID No.15, SEQ ID No.17 and SEQ ID No.19 respectively; and with corresponding nucleotide sequences encoding the amino acid sequence shown in SEQ ID No.12, SEQ ID No.14, SEQ ID No.16, SEQ ID No.18 and SEQ ID No.20 respectively) were 2.5×10⁹ VP/mouse/time or 5×10⁹ VP/mouse/time. Blood samples were harvested through orbital vein on day 35 (14 days after secondary immunization) and centrifuged at 6000 rpm for 10 minutes at 4°C. The serum samples were stored at -20°C before use, and subsequently, binding antibodies and neutralizing antibodies in the serum were detected to evaluate the immune effect of the vaccine. The mice were killed on day 49, and repeatedly flushed with 1 mL normal saline twice to harvest a bronchoalveolar lavage fluid. Subsequently, the binding antibodies and neutralizing antibodies in the bronchoalveolar lavage fluid were detected to evaluate the immune effect of the vaccine.
2. Immunization procedure of intranasal immunization adenovirus vector vaccine: female BALB/c mice aged 6-8 weeks were purchased from Charles River Laboratories, and the mice were housed under specific pathogen-free (SPF) conditions in National Key Laboratory of Biotherapy, Sichuan University. Mice were intranasally immunized twice on days 0 and 21, and the immunizing doses of used adenovirus vector RSV vaccines R1, R2, R3, R4 and R5 (with amino acid sequences shown in SEQ ID No.11, SEQ ID No.13, SEQ ID No.15, SEQ ID No.17 and SEQ ID No.19 respectively; and with corresponding nucleotide sequences encoding the amino acid sequence shown in SEQ ID No.12, SEQ ID No.14, SEQ ID No.16, SEQ ID No.18 and SEQ ID No.20 respectively) were 2.5×10⁹ VP/mouse/time or 5×10⁹ VP/mouse/time. Blood samples were harvested through orbital vein on day 35 (14 days after secondary immunization) and centrifuged at 6000 rpm for 10 minutes at 4°C. The serum samples were stored at -20°C before use, and subsequently, binding antibodies and neutralizing antibodies in the serum were detected to evaluate the immune effect of the vaccine. The mice were killed on day 49, and repeatedly whipped with 1 mL normal saline twice to harvest a bronchoalveolar lavage fluid. Subsequently, the binding antibodies and neutralizing antibodies in the bronchoalveolar lavage fluid were detected to evaluate the immune effect of the vaccine.
3. Immunization procedure of rats: female rats aged 6-8 weeks were purchased from Charles River Laboratories, and the rats were housed under specific pathogen-free (SPF) conditions in National Key Laboratory of Biotherapy, Sichuan University. The rats were intranasally immunized twice on days 0 and 21, and the immunizing doses of used adenovirus vector RSV vaccines R1, R2, R3, R4 and R5 (with amino acid sequences shown in SEQ ID No.11, SEQ ID No.13, SEQ ID No.15, SEQ ID No.17 and SEQ ID No.19 respectively; and with corresponding nucleotide sequences encoding the amino acid sequence shown in SEQ ID No.12, SEQ ID No.14, SEQ ID No.16, SEQ ID No.18 and SEQ ID No.20 respectively) were 2×10¹⁰ VP/ rat /time or 4×10¹⁰ VP/rat/time. Blood samples were harvested through orbital vein on day 35 (14 days after secondary immunization) and centrifuged at 6000 rpm for 10 minutes at 4°C. The serum samples were stored at -20°C before use, and subsequently, binding antibodies and neutralizing antibodies in the serum were detected to evaluate the immune effect of the vaccine.
4. Immunization procedure of challenge cotton rats: female cotton rats aged 6-8 weeks were purchased from SPF Biotechnology Co., Ltd., and the cotton rats were housed under specific pathogen-free (SPF) conditions in National Key Laboratory of Biotherapy, Sichuan University. The cotton rats were intranasally immunized twice on days 0 and 21, and the immunizing dose of the used adenovirus vector RSV vaccine R2 (with the amino acid sequence shown in SEQ ID No.13; and with the corresponding nucleotide sequence encoding the amino acid sequence shown in SEQ ID No.14) was 2.5×10⁹ VP/rat/time or 5×10⁹ VP/rat/time, and the cotton rats were challenged on day 35 (14 days after secondary immunization) to evaluate the immunoprotection effect of the vaccine.

### Embodiment 3 Detection of antibodies by enzyme-linked immunosorbent assay (ELISA)

In order to detect specific IgG and IgA against RSV preF (preF) and postF (postF), 1 µg/mL RSV preF (Sino Biological Inc.) or RSV postF (Sino Biological Inc.) solution was prepared with a 50 mM carbonate coating buffer (pH 9.6), and the prepared solution was added to a 96-well coating plate at 100 µL/well (Thermo Scientific Company, NUNC-MaxiSorp) and the plate was incubated overnight at 4°C. Preparation of 50 mM carbonate coating buffer (pH 9.6): 0.15 g Na₂CO₃ and 0.293 g NaHCO₃ were weighed and dissolved in double distilled water, and the prepared buffer was adjusted to pH 9.6, brought to a final volume of 100 mL, and stored at 4°C for later use.

The next day, the coating plate was washed three times with a PBS solution (PBST) including 0.1% Tween 20, then sealed at room temperature for 1 hour with a blocking buffer (prepared in PBST) including 1% BSA or 5% skim milk, and then washed once with PBST. The mouse serum or bronchoalveolar lavage fluid was diluted to different proportions with the blocking buffer, then the diluent was added into the coating plate at 100 µL/well, and the plate was incubated at 37°C for 1-2 hours and then washed three times with PBST.

Then, HRP-goat anti-mouse IgG or HRP-anti-mouse IgA antibody (both which were diluted in the blocking buffer at 1:10000) was added into the coating plate at 100 µL/well, and the plate was incubated at 37°C for 1 hour, and then washed five times with PBST. Finally, 3,3',5,5'-tetramethylbenzidine (TMB) was added into the coating plate at 100 µL/well; after the coating plate was subjected to color development for 10-15 minutes in the dark, 1 M H₂SO₄ stop solution (preparation of 1 H₂SO₄ stop solution: 2.7 mL of concentrated sulfuric acid (98%) was dropwise added into 47.3 mL of double distilled water) was added into the coating plate at 50 µL/well, mixed well and then read at a wavelength of 450 nm on a microplate reader.

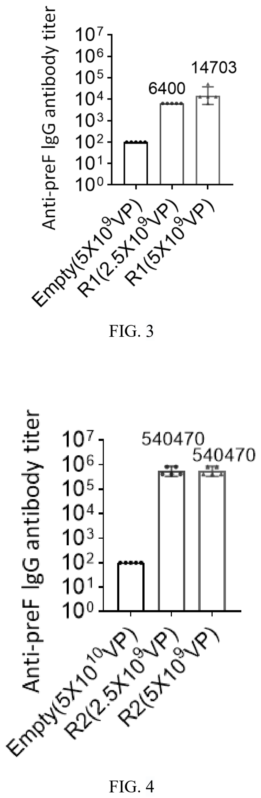

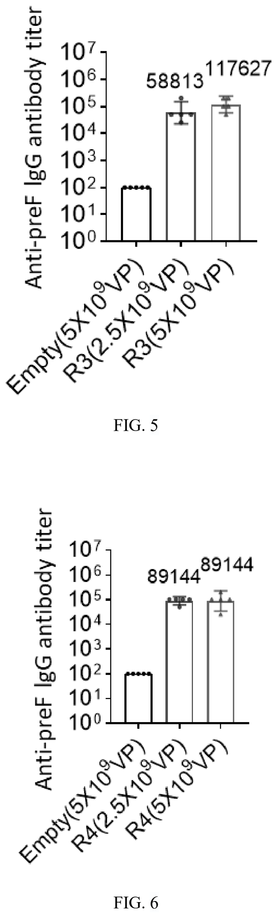

Results are as shown in FIG. 3-FIG.7. After the mice were subjected to two-dose intranasal immunization, different doses of adenovirus vector RSV vaccines (R1, R2, R3, R4, R5) could induce high levels of anti-preF IgG in serum, and especially the level of anti-preF IgG induced by the adenovirus vector RSV vaccine R2 (with amino sequence shown in SEQ ID No.13, nucleotide sequence shown in SEQ ID No.14, RSV-A-PreF-1F (R2) (Del104-145aa))was significantly higher than that of other intranasal adenovirus vector RSV vaccines, indicating that R2 could have potent humoral immunoprotective efficacy against RSV.

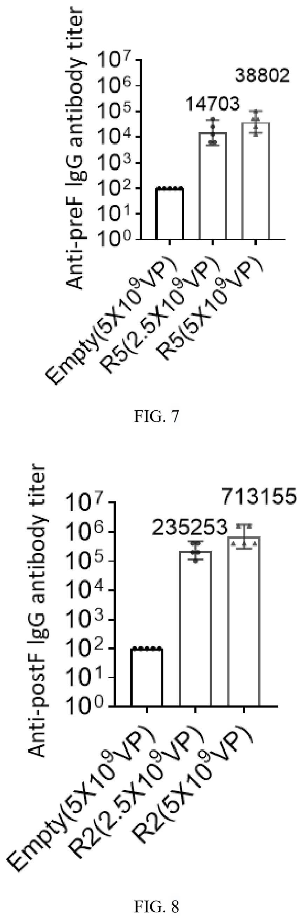

Results are as shown in FIG. 8. After the mice were subjected to two-dose intranasal immunization, different doses of adenovirus vector RSV vaccine R2 could induce anti-postF IgG in serum, indicating that R2 could have potent humoral immunoprotective efficacy against RSV.

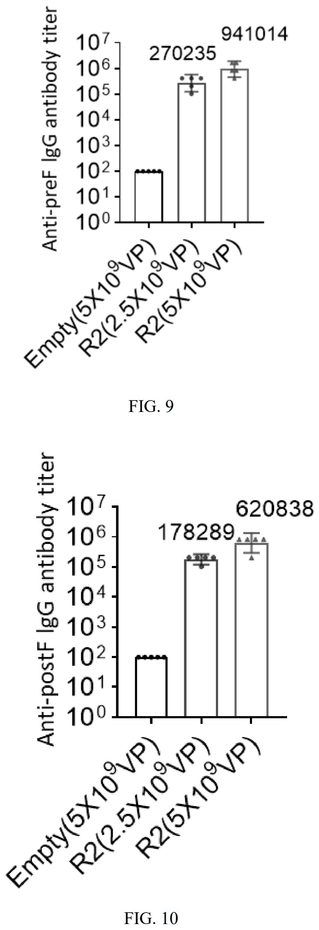

Results are as shown in FIG. 9 and FIG. 10. After the mice were subjected to two-dose intramuscular immunization, different doses of adenovirus vector RSV vaccine R2 could induce anti-preF and anti-postF IgG in serum, indicating that R2 could have potent humoral immunoprotective efficacy against RSV.

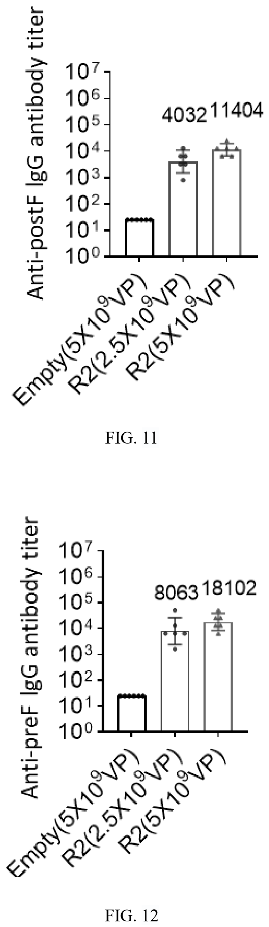

Results are as shown in FIG. 11 and FIG. 12. After the mice were subjected to two-dose intranasal immunization, the adenovirus vector RSV vaccine R2 could induce anti-preF and anti-postF IgG in the bronchoalveolar lavage fluid, indicating that R2 could have potent in-situ humoral immunoprotective efficacy against RSV.

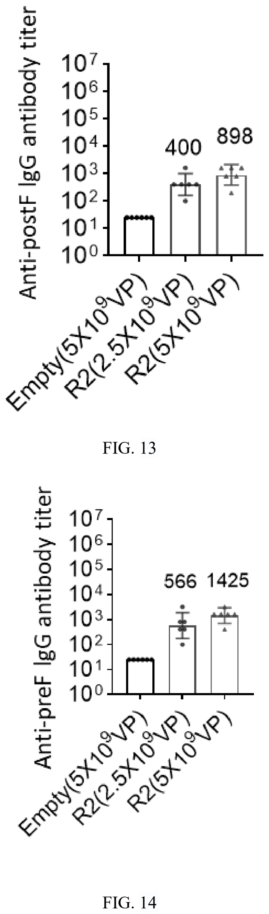

Results are as shown in FIG. 13 and FIG. 14. After the mice were subjected to two-dose intranasal immunization, the adenovirus vector RSV vaccine R2 could induce anti-preF and anti-postF IgA in the bronchoalveolar lavage fluid, indicating that R2 could have potent in-situ humoral immunoprotective efficacy against RSV.

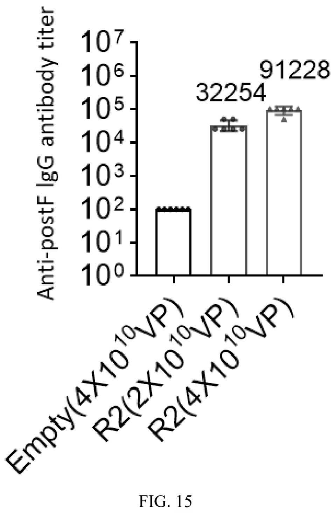

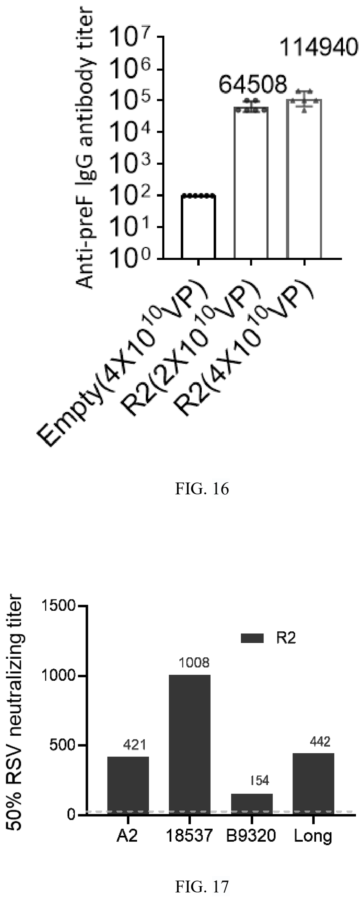

Results are as shown in FIG. 15 and FIG. 16. After the rats were subjected to two-dose intranasal immunization, the adenovirus vector RSV vaccine R2 could induce anti-preF and anti-postF IgG in serum, indicating that R2 could have potent humoral immunoprotective efficacy against RSV.

### Embodiment 4 RSV neutralizing antibody detection experiment

In order to detect the titer of neutralizing antibodies in serum and bronchoalveolar lavage fluid (BALF) samples, RSV neutralizing antibody detection experiments were performed. RSV A2, Long, B18357 and B9320 strains were purchased from ATCC Company of the United States.

Cell culture: A549 cells were resuspended in a complete DMEM medium (DMEM culture medium including 10% fetal bovine serum and 1000 IU penicillin/streptomycin) at a cell density of 4×10⁵/mL. Then, 100 µL of the cell suspension was added to a 96-well flat-bottom sterile plate, and the cells were cultured overnight in an incubator (5% CO₂) at 37°C.

Positive control: the positive serum was inactivated in a water bath at 56°C for 30 minutes before use, and diluted 100 folds with DMEM medium without fetal bovine serum, and then 110 µL of positive serum diluted 100 folds was added into A12 well. 55 µL of DMEM culture medium without fetal bovine serum was added into B12-H12 wells; 55 µL of liquid was transferred from A12 well to B12 well, and mixed well by gently pipetting up and down 6-8 times; such a transfer process was repeatedly performed until the liquid was transferred to H12 well, and finally 55 µL of liquid was aspirated and discarded from H12 well.

Sample dilution: the samples were initially diluted 30 folds in triplicate, and the samples with 7 concentration gradients were diluted by a 2-fold dilution method. The initially diluted samples were added into A1-A9 wells, and the sample well volume before gradient dilution was 110 µL/well. 55 µL of DMEM culture medium without fetal bovine serum was added into A2-H11 wells; the multi-channel pipette was adjusted to 55 µL, and the liquid in A1-A9 wells was gently pipetted up and down 6-8 times and mixed well; 55 µL of liquid was transferred to corresponding B1-B9 wells, and gently pipetted up and down 6-8 times and then transferred into D1-D9 wells; such a transfer process was repeatedly performed, and finally 50 µL of liquid was aspirated and discarded from H1-H9 wells.

Incubation of samples with virus: 55 µL of RSV virus was added to each well in columns 1-10 and 12, so that the load of pseudovirus in each well was 200 PFU/well. Column 10 represents the positive control group. 55 µL DMEM culture medium without fetal bovine serum was added into column 11, and the column 11 represents the negative control group. The 96-well plate was incubated in a cell incubator (37°C, 5% CO₂) for 1 hour.

Inoculation of cell virus: the cultured 96-well plate including A549 cells was placed upside down on sterile absorbent paper, the supernatant was aspirated, PBS was added and aspirated twice to prevent the cells from being washed away, the final PBS was aspirated and discarded, 100 µL of the mixture of virus and serum was added into a cell well plate, and the plate was incubated in an incubator at 37°C for 1 hour. After that, 90 µL of liquid was aspirated out of each well with a multi-channel pipette, and 100 µL of complete culture medium was added into each well and the plate was incubated in an incubator at 37°C for 3 days.

Fixation: the supernatant was discarded by gently turning the plate, and 200 µL of fixation buffer (80% acetone, 20% PBS) was added to each well to fix the cells. The plate was incubated for 20 minutes at -20°C. The fixation buffer was discarded and the well plate was allowed to face down to dry for 10 minutes. 200 µL of blocking buffer (5% milk diluent prepared by PBST) was added to each well, and the plate was incubated at room temperature for 30 minutes. The blocking buffer was discarded by gently turning the plate over; goat anti-RSV antibody (primary antibody, 1:500) diluted with 50 µL/well blocking buffer was added, and the plate was incubated at 37°C for 1 hour, and washed three times with PBST. After that, Alexa-Fluor donkey anti-goat IgG antibody (secondary antibody, 1:50 000) diluted with 50 µL blocking buffer per well was added, and the plate was incubated at 37°C for 1 hour and washed five times with PBST.

Reading: read plates using a fluorescein isothiocyanate (FITC) channel with counting settings on an automated spot reader.

Determination of 50% neutralization titer: inhibition rate = [1-(mean of luminous intensity of sample group-mean of negative control group)/ (mean of luminous intensity of positive group-mean of negative control group CC)] × 100%. According to the results of neutralization inhibition rate, EC50 (IC50) was calculated by Reed-Muench method.

Results are as shown in FIG. 17. After the mice were subjected to two-dose intranasal immunization (5×10⁹ VP/mouse/time), the adenovirus vector RSV vaccine R2 could induce neutralizing antibodies against A2, B18537, B9320 and Long, indicating that R2 intranasal immunization could induce broad spectrum and potent immunoprotection.

### Embodiment 5 RSV challenge experiment

On days 0 and 14, cotton rats were intranasally immunized with a low dose (2.5×10⁹ VP/rat) and high dose (5×10⁹ VP/rat) of adenovirus vector vaccine R2. The cotton rats immunized with adenovirus empty vector vaccine served as the control group, and n=5 rats per group. Intranasal immunization was performed with a volume of 50 µL/mouse, administered in two divided doses with a 2-hour interval. On day 35, a challenge experiment was performed. The mice were challenged intranasally with a volume of 100 µL/mouse and with the dosage of 3×10⁶ PFU of RSV-A2 or RSV-B18537 strain. Cotton rats were euthanized five days after infection; the left lung lobe was fixed, sliced, and stained with HE for pathological examination, and the right lung lobe was taken for virus load detection; and the tissue was cut into pieces and homogenized, and about 100 mg of the tissue was weighed and homogenized in 800 µL of TRIzol reagent. 400 µL of tissue homogenate was taken to extract RNA, and RNA was finally dissolved in 50 µL of nuclease-free solution and stored at -80°C for later use. RNA was used for the subsequent detection of viral load by qRT-PCR (one-step method). The genomic RNA (gRNA) and subgenomic RNA (sgRNA) of the virus were determined by real-time quantitative reverse transcription PCR (qRT-PCR). Primer and probe sequences used for gRNA are derived from N gene, primer sequences: 5'-CTCCTAATTATGATGTGCAGAAACACA-3' (SEQ ID No. 37 forward), 5'-CCAGTGAATTTATGATTAGCATCTTCT-3' (SEQ ID No.38 reverse); probe sequence 5'-FAM-ATAACATGCCACATAACTTA-BHQ1-3' (SEQ ID No. 39).

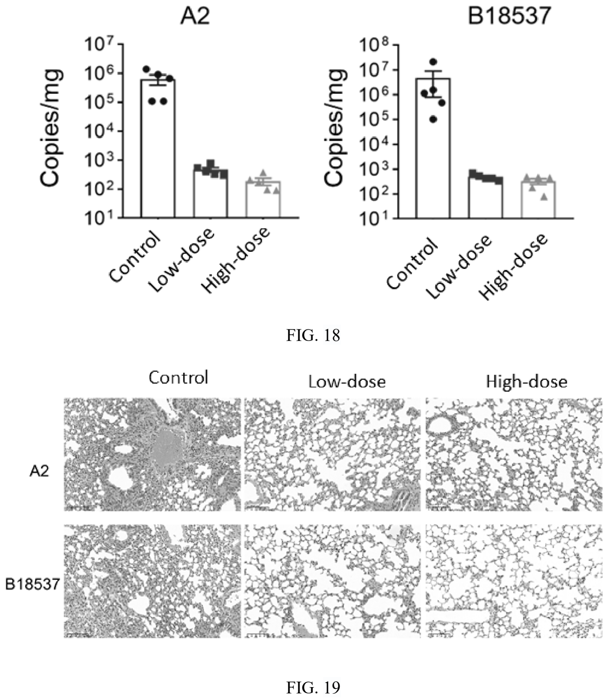

Pulmonary viral load and pathological changes after respiratory syncytial virus infection are important indexes to evaluate the protective efficacy of respiratory syncytial virus vaccine candidates. Cotton rats were challenged with live RSV-A2 and RSV-B18537 strains (3×10⁶ PFU/cotton rat) after being vaccinated with two doses of vaccine. Animals were euthanized on day 5 after infection, and the right lung lobe was taken for virus load detection (FIG. 18), and the left lung lobe was fixed, sliced, and stained with HE for pathological examination (FIG. 19). The results showed that after the two virus strains were challenged, the virus load in the lung tissue of the experimental animals in the empty load control group was about 10⁶ copies/mg tissue, and the virus load in the lung tissue of the animals in the vaccine immunization group decreased to below 10³ copies/mg tissue (FIG. 18), demonstrating that vaccine immunization could effectively neutralize and eliminate the virus in the lung tissue of cotton rats. Pathological results showed that the lung tissue of the animals in the empty load group presented obvious alveolar wall thickening, accompanied by local hemorrhage and infiltration by a large number of inflammatory cells. The pathological conditions of the lung tissues of the animals in the vaccine immunization group were significantly improved. The alveolar contours were clear, with infiltration by only a small amount of inflammatory cell infiltration (FIG. 19). These results showed that vaccine immunization provided cotton rats with substantial protection against RSV-induced diseases.

## Claims

1. An adenovirus vector vaccine for preventing and/or treating respiratory syncytial virus infection, wherein the adenovirus vector vaccine is obtained by constructing a recombinant adenovirus vector comprising a respiratory syncytial virus expression gene, and a polynucleotide sequence of the respiratory syncytial virus expression gene is selected from at least one of SEQ ID No.12, SEQ ID No.14, SEQ ID No.16, SEQ ID No.18, SEQ ID No.20, SEQ ID No.22, SEQ ID No.24, SEQ ID No.26, SEQ ID No.28, SEQ ID No.30, SEQ ID No.32 and SEQ ID No.34.

2. The adenovirus vector vaccine according to claim 1, wherein an amino acid sequence of a protein encoded by the polynucleotide sequence is selected from at least one of SEQ ID No.11, SEQ ID No.13, SEQ ID No.15, SEQ ID No.17, SEQ ID No.19, SEQ ID No.21, SEQ ID No.23, SEQ ID No.25, SEQ ID No.27, SEQ ID No.29, SEQ ID NO.31 and SEQ ID No.33.

3. The adenovirus vector vaccine according to claim 1 or 2, further comprising pharmaceutically acceptable adjuvants, vectors, diluents or excipients.

4. The adenovirus vector vaccine according to claim 1 or 2, wherein the adenovirus vector is selected from at least one of the following: adenovirus, Ankara vaccinia virus and adeno-associated virus; preferably, replication-deficient adenovirus of human type 5, 35 or 26 or/and chimpanzee type AdC68 or AdC7; and more preferably, human type 5 replication-deficient adenovirus with combined deletion of E1 and E3.

5. The adenovirus vector vaccine according to any one of claims 1 to 4, wherein the vaccine is formulated as an intradermal injection preparation or a subcutaneous injection preparation, an intramuscular injection preparation, an intravenous injection preparation, an oral preparation or a nasal spray preparation; and preferably, the vaccine is formulated as the nasal spray preparation or the intramuscular injection preparation.

6. A method for preparing an adenovirus in the adenovirus vector vaccine according to any one of claims 1 to 5, comprising the following steps: constructing a shuttle plasmid vector comprising polynucleotide; transfecting the constructed shuttle plasmid vector and backbone plasmid into a host cell to culture the host cell; obtaining a replication-deficient recombinant adenovirus; and performing large-scale cultivation and purification.

7. A composition for preventing and/or treating respiratory syncytial virus infection, wherein the composition is a compound preparation comprising a recombinant protein vaccine for preventing and/or treating the respiratory syncytial virus infection and the adenovirus vector vaccine according to any one of claims 1 to 5 as active components.

8. A combined drug for preventing and/or treating respiratory syncytial virus infection, comprising a recombinant protein vaccine for preventing and/or treating the respiratory syncytial virus infection and the adenovirus vector vaccine according to any one of claims 1 to 5, wherein these vaccines are administered separately or simultaneously.

9. The composition according to claim 7 or the combined drug according to claim 8, wherein the recombinant protein vaccine comprises the recombinant protein for preventing and/or treating the respiratory syncytial virus infection, and the amino acid sequence of the recombinant protein has more than 70% sequence identity and the same or substantially equivalent biological activity with SEQ ID No.1 or SEQ ID No.2; and preferably, the amino acid sequence of the recombinant protein is selected from at least one of SEQ ID No.1, SEQ ID No.2, SEQ ID No.3, SEQ ID No.4, SEQ ID No.5 and SEQ ID NO.6.

10. The composition or combined drug according to claim 9, wherein the recombinant protein vaccine comprises a protein precursor, and the protein precursor is constructed by at least one amino acid sequence selected from SEQ ID No.7 and SEQ ID No.8; and preferably, a nucleotide sequence encoding the protein precursor is selected from SEQ ID No.9 or SEQ ID No.10.

11. The composition according to any one of claims 7, 9 and 10 or the combined drug according to any one of claims 8 to 10, wherein the composition or the combined drug is formulated as an intradermal injection preparation or a subcutaneous injection preparation, an intramuscular injection preparation, an intravenous injection preparation, an oral preparation or a nasal spray preparation; and preferably, the vaccine is formulated as the nasal spray preparation.

12. Use of the adenovirus vector vaccine according to any one of claims 1 to 5, the composition according to claims 7, 9 to 11, or the combined drug according to any one of claims 8 to 11 in preparing drugs for preventing and/or treating respiratory syncytial virus infection.
